(19) Europäisches Patentamt / European Patent Office / Office européen des brevets

(11) **EP 2 709 973 B1**

(12) **FASCICULE DE BREVET EUROPEEN**


(45) Date de publication et mention de la délivrance du brevet:
**21.09.2016 Bulletin 2016/38**

(21) Numéro de dépôt: **12720510.2**

(22) Date de dépôt: **15.05.2012**

(51) Int Cl.:
***C07C 37/60*** *(2006.01)* ***C07C 39/08*** *(2006.01)*

(86) Numéro de dépôt international:
**PCT/EP2012/058961**

(87) Numéro de publication internationale:
**WO 2012/156381 (22.11.2012 Gazette 2012/47)**


(54) **PROCEDE D'HYDROXYLATION DE PHENOLS ET D'ETHERS DE PHENOLS**

VERFAHREN ZUR HYDROXYLIERUNG VON PHENOLEN UND PHENOLETHERN

METHOD FOR HYDROXYLATING PHENOLS AND PHENOL ETHERS


(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.05.2011 FR 1154372**

(43) Date de publication de la demande:
**26.03.2014 Bulletin 2014/13**



(73) Titulaire: **Rhodia Operations**
**75009 Paris (FR)**

(72) Inventeurs:
- **GAREL, Laurent**
  **F-69003 Lyon (FR)**
- **NORMAND, Stéphanie**
  **F-69230 Saint-Genis-Laval (FR)**
- **PITIOT, Pascal**
  **F-69008 Lyon (FR)**
- **BIGOURAUX, Jean-Christophe**
  **F-42800 Dargoire (FR)**

(74) Mandataire: **Menville, Laure et al**
**Rhodia Opérations**
**IAM**
**85, avenue des frères Perret**
**69192 Saint-Fons (FR)**



(56) Documents cités:
**WO-A2-2009/024446 DE-A1- 10 320 634**
**DE-A1- 19 835 907**

- **YUBE ET AL: "Control of selectivity in phenol hydroxylation using microstructured catalytic wall reactors", APPLIED CATALYSIS A: GENERAL, ELSEVIER SCIENCE, AMSTERDAM, NL, vol. 327, no. 2, 26 juillet 2007 (2007-07-26), pages 278-286, XP022146424, ISSN: 0926-860X, DOI: 10.1016/J.APCATA.2007.05.026**
- **DATABASE CA [Online] CHEMICAL ABSTRACTS SERVICE, COLUMBUS, OHIO, US; NAGAI, SHIGEKI ET AL: "Dihydric phenols", XP002667323, extrait de STN Database accession no. 1975:513953 & JP 50 062940 A 29 mai 1975 (1975-05-29)**
- **CENTI G ET AL: "One-step H2O2 and phenol syntheses: Examples of challenges for new sustainable selective oxidation processes", CATALYSIS TODAY, ELSEVIER, NL, vol. 143, no. 1-2, 15 May 2009 (2009-05-15), pages 145-150, XP026050563, ISSN: 0920-5861, DOI: 10.1016/J.CATTOD.2008.09.032 [retrieved on 2008-11-18]**



Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).





Printed by Jouve, 75001 PARIS (FR)

## Description

La présente invention a pour objet un procédé d'hydroxylation de phénols et d'éthers de phénols par le peroxyde d'hydrogène.

L'invention vise plus particulièrement un procédé d'hydroxylation du phénol par le peroxyde d'hydrogène.

Dans l'exposé qui suit de la présente invention, le terme « substrat phénolique » est utilisé pour désigner indifféremment un phénol ou un éther de phénol.

La réaction d'hydroxylation du phénol par le peroxyde d'hydrogène conduit à l'obtention de deux isomères à savoir le 1,4-dihydroxybenzène ou hydroquinone (HQ) et le 1,2-dihydroxybenzène ou pyrocatéchol (PC).

Dans le présent texte, on désigne par « diphénol », l'hydroquinone et le pyrocatéchol.

L'hydroquinone est un produit utilisé dans de nombreux domaines d'application en tant qu'inhibiteur de polymérisation, anti-oxydant dans les élastomères ou comme intermédiaire de synthèse. Un autre domaine d'application est la photographie.

Le pyrocatéchol est également un produit largement utilisé notamment comme inhibiteur de polymérisation ou anti-oxydant dans les élastomères, oléfines, polyoléfines ou polyuréthane ou comme agent tannant.

Le pyrocatéchol, en raison de ses propriétés complexantes, est également employé comme agent chélatant notamment dans le domaine de l'électronique et comme inhibiteur de corrosion.

Il sert également comme intermédiaire dans de nombreuses synthèses notamment celles des parfums, cosmétiques, médicaments, pesticides.

Il s'ensuit que l'hydroquinone et le pyrocatéchol sont des produits de grande consommation fabriqués à grande échelle.

Ainsi, compte tenu de l'importance des volumes de fabrication, il importe que leur procédé de fabrication soit optimisé au maximum en particulier en termes de productivité, d'efficacité énergétique et de rendement.

L'hydroquinone et le pyrocatéchol sont produits classiquement par hydroxylation du phénol par le peroxyde d'hydrogène, en présence d'un catalyseur acide, acide protonique fort ou bien un catalyseur solide à propriétés acides comme par exemple la TS-1.

APPLIED CATALYSIS A: GENERAL, vol. 327, no. 2, 2007, pages 278-286 divulgue un procédé d'hydroxylation du phénol comprenant une première étape de mélange du phénol avec une solution de peroxyde d'hydrogène, une deuxième étape consistant à effectuer la réaction dans un micro-réacteur dans des conditions adiabatiques et une troisième étape de récupération du phénol, de l'hydroquinone, du catéchol et de la benzoquinone.

CATALYSIS TODAY, vol. 143, 2009, pages 145-150 enseigne un procédé d'hydroxylation du benzène comprenant une première étape de mélange du benzène avec une solution de peroxyde d'hydrogène, une deuxième étape consistant à effectuer dans des conditions adiabatiques et une troisième étape de récupération de DHB.

JP 50 062940 A divulgue un procédé d'hydroxylation du phénol.

L'une des voies bien connue de préparation desdits diphénols consiste, selon FR 2 071 464, à effectuer l'hydroxylation du phénol par le peroxyde d'hydrogène, en présence d'un acide protique fort tel que par exemple, l'acide sulfurique, l'acide chlorosulfurique, l'acide perchlorique, les acides sulfoniques comme par exemple, l'acide méthanesulfonique, trifluorométhanesulfonique, toluènesulfonique, phénolsulfonique.

Le peroxyde d'hydrogène est mis en oeuvre sous forme d'une solution aqueuse.

La solution de peroxyde d'hydrogène classiquement commercialisée a une concentration de l'ordre de 30 % ce qui induit des inconvénients au niveau de la réaction car la présence d'eau ralentit la réaction et au niveau du bilan énergétique puisque cette eau doit être ensuite éliminée.

Le recours à des solutions plus concentrées de peroxyde d'hydrogène est souhaitable mais leur utilisation à l'échelle industrielle est délicate car les risques d'explosivité sont d'autant plus accrus que la concentration en peroxyde d'hydrogène est élevée.

Par ailleurs, le phénol est toujours mis en oeuvre en large excès par rapport à la quantité de peroxyde d'hydrogène, Ainsi, le rapport molaire peroxyde d'hydrogène/phénol varie généralement entre 0,01 et 0,3.

La présence d'un fort excès de phénol implique en fin de réaction, la nécessité de le séparer du milieu réactionnel afin de le recycler.

Ce surcoût est d'autant plus réduit que le taux de transformation du phénol est élevé.

Toutefois, lorsque la réaction d'hydroxylation du phénol est conduite dans un dispositif de mélange, classiquement utilisé tel que réacteur agité ou cascade de réacteurs agités, le taux de transformation du phénol est maintenu peu élevé (moins de 5%) pour garantir de bonnes performances réactionnelles. Lorsque le taux de transformation du phénol est augmenté pour atteindre 15 - 20 %, les rendements en diphénols obtenus sont divisés par deux. En effet, le rendement baisse car le taux de sous-produits augmente par dégradation des diphénols notamment, en raison de réactions d'oxydation consécutives.

Il est donc très intéressant d'accroître le taux de transformation du phénol tout en maintenant la sélectivité et/ou le rendement en hydroquinone et pyrocatéchol.

Ainsi, l'objectif de la présente invention est de fournir un procédé amélioré de préparation d'hydroquinone et de pyrocatéchol en termes de bilan matières et d'efficacité énergétique.

Il a maintenant été trouvé et c'est ce qui constitue l'objet de la présente invention, un procédé d'hydroxylation d'un phénol ou d'un éther de phénol, par réaction dudit phénol ou éther de phénol, avec le peroxyde d'hydrogène, en présence d'un catalyseur acide fort homogène avant un pKa dans l'eau inférieur à -0,1, caractérisé par le fait qu'il comprend les étapes suivantes mises en oeuvre successivement ou simultanément :

- une première étape de mélange d'un phénol ou éther de phénol avec une solution de peroxyde d'hydrogène dans des conditions telles que la température soit suffisante pour que le phénol ou l'éther de phénol de départ reste liquide et que le taux de transformation du peroxyde d'hydrogène soit inférieur à 25 % en mol,
- une deuxième étape consistant à effectuer la réaction d'hydroxylation du phénol ou de l'éther de phénol dans des conditions adiabatiques ; le catalyseur acide étant introduit à l'étape du mélange et/ou en début de réaction d'hydroxylation,
- une troisième étape si nécessaire de récupération du produit hydroxylé.

Dans le présent texte, on entend par « conditions adiabatiques », le fait de conduire la réaction dans une enceinte adiabatique c'est-à-dire dans une enceinte isolée du milieu extérieur de telle sorte que la réaction soit conduite sans apport énergétique extérieur. En d'autres termes, la réaction d'hydroxylation a lieu sans que la température de la réaction soit régulée par un échange thermique extérieur.

Jusqu'à présent, il n'a jamais été décrit qu'une réaction d'hydroxylation pouvait avoir lieu dans des conditions adiabatiques. En particulier, une réaction d'hydroxylation en milieu comprenant une seule phase liquide n'a jamais été divulguée dans l'état de la technique.

Conformément au procédé de l'invention, il a été trouvé que le fait de conduire la réaction d'hydroxylation dans des conditions adiabatiques permettait de réduire le temps de réaction, à conversion et sélectivité données. Ceci conduit donc à un accroissement significatif de la productivité de l'appareillage avec, par exemple, en mode continu, une réduction du temps de passage du milieu réactionnel ou une diminution du volume de l'appareillage

Les exemples de réalisation décrits plus loin dans le texte attestent des gains obtenus.

Conformément au procédé de l'invention, on fait réagir le composé phénolique avec le peroxyde d'hydrogène en présence d'un catalyseur et éventuellement d'un co-catalyseur.

Le procédé de l'invention est bien adapté à l'hydroxylation du phénol ou d'un éther de phénol mais également aux phénols ou éthers de phénols substitués.

Dans le présent texte, le terme « substrat phénolique » ou « composé phénolique » est utilisé pour désigner le phénol, les phénols et les éthers phénoliques.

Par « phénol ou éther de phénol substitué », on entend un phénol ou un éther de phénol dont l'un des atomes d'hydrogène du cycle aromatique est remplacé par un ou plusieurs substituants.

Généralement, par plusieurs substituants, on définit moins de quatre substituants par noyau aromatique.

N'importe quel substituant peut être présent dans la mesure où il n'interfère pas dans la réaction de l'invention.

Ainsi, le procédé de l'invention est bien adapté pour s'appliquer aux substrats phénoliques de formule générale (I) :

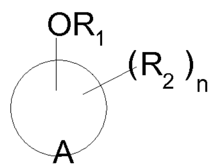

(I)

dans laquelle :

- A symbolise un cycle benzénique ou naphtalénique,
- $R_1$ représente un atome d'hydrogène, un groupe alkyle, cycloalkyle, aryle, aralkyle,
- $R_2$ représente un atome d'hydrogène ou un ou plusieurs substituants, identiques ou différents,
- n, nombre de substituant par cycle aromatique, est un nombre inférieur ou égal à 4.

Dans la formule (I), le groupe $OR_1$ est un groupe éther dès lors que $R_1$ est différent d'un atome d'hydrogène.

Le nombre de substituants par cycle aromatique est variable et généralement inférieur ou égal à 4, de préférence égal à 0, 1, 2 ou 3.

Des exemples préférés de substituants sont donnés pour la formule (Ia).

Ainsi, le procédé de l'invention est bien adapté aux substrats phénoliques répondant à la formule (I) dans laquelle A représente un cycle benzénique et qui sont représentés plus particulièrement par la formule générale (Ia) :

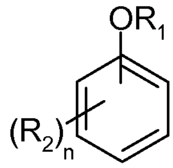

(Ia)

dans ladite formule :

- n est un nombre de 0 à 4, de préférence égal à 0, 1 ou 2,
- $R_1$ représente un atome d'hydrogène, un groupe alkyle, cycloalkyle, aryle, aralkyle,
- $R_2$, identiques ou différents, représentent un groupe alkyle, un groupe alkoxy, un groupe hydroxyle, un atome d'halogène, un groupe halogéno-ou perhalogénoalkyle.

Le procédé de l'invention s'applique préférentiellement aux substrats répondant à la formule (Ia) dans laquelle n est égal à 0 ou 1 ; $R_1$ représente un atome d'hydrogène, un groupe alkyle ayant de 1 à 4 atomes de carbone ; $R_2$ représente un atome d'hydrogène, un groupe alkyle ou alkoxy ayant de 1 à 4 atomes de carbone.

Dans les formules (I) et (Ia), on entend par « alkyle », une chaîne hydrocarbonée linéaire ou ramifiée en $C_1$-$C_{15}$, de préférence en $C_1$-$C_{10}$ et encore plus préférentiellement en $C_1$-$C_4$. Des exemples de groupes alkyle préférés sont notamment méthyle, éthyle, propyle, isopropyle, butyle, isobutyle, t-butyle.

Par « alkoxy », on entend un groupe alkyl-O- dans lequel le terme alkyle a la signification donnée ci-dessus. Des exemples préférés de groupes alkoxy sont les groupes méthoxy ou éthoxy.

Par « cycloalkyle », on entend un groupe hydrocarboné cyclique, monocyclique en $C_3$-$C_8$, de préférence, un groupe cyclopentyle ou cyclohexyle.

Par « aryle », on entend un groupe mono- ou polycyclique aromatique, de préférence, mono- ou bicyclique en $C_6$-$C_{20}$, de préférence, phényle ou naphtyle. Lorsque le groupe est polycyclique c'est-à-dire qu'il comprend plus d'un noyau cyclique, les noyaux cycliques peuvent être condensés deux à deux ou rattachés deux à deux par des liaisons $\sigma$. Des exemples de groupes ($C_6$-$C_{18}$)aryle sont notamment phényle, naphtyle.

Par « aralkyle », on entend un groupe hydrocarboné, linéaire ou ramifié porteur d'un cycle aromatique monocyclique en $C_7$-$C_{12}$, de préférence, benzyle : la chaîne aliphatique comprenant 1 ou 2 atomes de carbone

Par « groupe halogénoalkyle », on entend un groupe alkyle tel que précédemment défini dont l'un ou plusieurs atomes d'hydrogène sont remplacés par un atome d'halogène, de préférence un atome de fluor.

Par « groupe perhalogénoalkyle », on entend un groupe alkyle comprenant de 1 à 10 atomes de carbone et de 3 à 21 atomes d'halogène de préférence de fluor et plus particulièrement le groupe trifluorométhyle.

Par « atome d'halogène », on définit le fluor, le chlore et le brome.

A titre illustratif de substrats phénoliques de formule (I) susceptibles d'être mis en oeuvre dans le procédé de l'invention, on peut mentionner plus particulièrement :

- ceux répondant à la formule (I) dans laquelle n est égal à 0, tels que le phénol ou l'anisole,
- ceux répondant à la formule (I) dans laquelle n est égal à 1, tels que l'ocrésol, le m-crésol, le p-crésol, le 2-éthylphénol, le 3-éthylphénol, 2-propylphénol, le 2-sec-butylphénol, le 2-tert-butylphénol, le 3-tert-butylphénol, le 4-tert-butylphénol, le 2-méthoxyphénol, le 3-méthoxyphénol, le 4-méthoxyphénol, le 2-éthoxyphénol, le salicylate de méthyle, le 2-chlorophénol, le 3-chlorophénol, le 4-chlorophénol,
- ceux répondant à la formule (I) dans laquelle n est égal à 2, tels que le 2,3-diméthylphénol, le 2,5-diméthylphénol, le 2,6-diméthylphénol, le 3,5-diméthyphénol, le 2,3-dichlorophénol, le 2,5-dichlorophénol, le 2,6-dichlorophénol, le 3,5-dichlorophénol, le 2,6-ditert-butylphénol, le 3,5-ditert-butylphénol,
- ceux répondant à la formule (I) dans laquelle n est égal à 3, tels que le 2,3,5-triméthylphénol, le 2,3,6-triméthylphénol, le 2,3,5-trichlorophénol, le 2,3,6-trichlorophénol,
- ceux répondant à la formule (I) dans laquelle A représente un cycle naphtalénique, tels que le 1-hydroxynaphtalène,

Parmi les substrats phénoliques précités, on met en oeuvre préférentiellement, le phénol, l'o-crésol, le m-crésol, le p-crésol, l'anisole, le phénétole, le 2-méthoxyphénol (le gaïacol), le 2-éthoxyphénol (le guétol).

Le présent procédé convient tout particulièrement bien à la préparation d'hydroquinone et de pyrocatéchol à partir du phénol.

Intervient dans le procédé de l'invention, un catalyseur homogène qui est un acide fort. Par acide fort, on désigne dans la présente invention, un acide présentant un pKa dans l'eau inférieur à - 0,1 et, de préférence, inférieur à-1,0.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de l'oxydation par le peroxyde d'hydrogène.

On peut citer plus particulièrement les oxyacides halogénés ou non tels que l'acide sulfurique, l'acide phosphorique, l'acide pyrosulfurique, l'acide perchlorique ; les acides sulfoniques aliphatiques ou aromatiques tels que par exemple, l'acide méthanesulfonique, l'acide éthanesulfonique, l'acide benzènesulfonique, l'acide bis-trifluorométhanesulfonimide, les acides toluènesulfoniques, les acides naphtalènesulfoniques, les acides benzènedisulfoniques, les acides naphtalènedisulfoniques; les acides halogénosulfoniques tels que l'acide fluorosulfonique, l'acide chlorosulfonique ou l'acide trifluorométhanesulfonique.

Parmi les acides précités, on utilise de préférence l'acide sulfurique, l'acide perchlorique, l'acide méthanesulfonique, trifluorométhanesulfonique, toluènesulfonique, phénolsulfonique, l'acide bis-trifluorométhanesulfonimide.

Selon une variante du procédé de l'invention, il est possible d'utiliser comme acide protonique fort, un acide hydroxyaromatique sulfonique tel que décrit dans WO 2009/150125.

Comme exemples préférés d'acides hydroxyaromatiques sulfoniques mis en oeuvre préférentiellement dans le procédé de l'invention, on peut mentionner les acides répondant à la formule suivante :

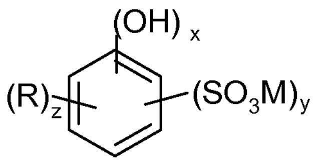

(Ia)

dans ladite formule :

- x est égal à 1,2 ou 3, de préférence 1 ou 2,
- y est égal à 1 ou 2,
- z est un nombre de 0 à 4, de préférence égal à 0, 1 ou 2,
- M représente un atome d'hydrogène, le sodium ou le potassium,
- R représente un groupe alkyle ou alkoxy ayant de 1 à 4 atomes de carbone, un groupe carboxylique.

Parmi les acides convenant au procédé de l'invention, on peut citer plus particulièrement, les acides hydroxybenzènesulfoniques, les acides hydroxybenzoïques sulfonés ; les acides hydroxybenzènedisulfoniques, les acides dihydroxybenzènedisulfoniques les acides hydroxytoluènesulfoniques, les acides hydroxynaphtalènesulfoniques et les acides hydroxynaphtalènedisulfoniques et leurs mélanges.

Parmi les acides hydroxybenzènesulfoniques, on utilisera de préférence l'acide 4-hydroxybenzènesulfonique, l'acide 2-hydroxybenzènesulfonique, l'acide 5-sulfosalicylique ou leur mélange.

Comme exemples préférés d'acides dihydroxybenzènesulfoniques mis en oeuvre, on peut citer les acides sulfoniques résultant de la sulfonation de l'hydroquinone (1,4-dihydroxybenzène), du pyrocatéchol (1,2-dihydroxybenzène), et de la résorcine (1,3-dihydroxybenzène).

Les acides dihydroxybenzènedisulfoniques préférés sont l'acide 5,6-dihydroxy-1,3-benzènedisulfonique, l'acide 4,6-dihydroxy-1,3-benzène-disulfonique, l'acide 2,5-dihydroxy-1,4-benzènedisulfonique.

Les acides hydroxyaromatiques sulfoniques sont disponibles sous forme solide, liquide ou en solution aqueuse dont la concentration peut varier entre 5 et 95 % en poids, de préférence entre 50 et 70 % en poids.

Selon une autre variante du procédé de l'invention, il est possible d'utiliser un mélange d'au moins deux acides protoniques forts, tel que décrit dans WO 2010/115784.

Le mélange comprend deux acides (A) et (B) ayant respectivement des pKa spécifiques : l'acide (B) étant beaucoup plus fort que l'acide (A).

Ledit mélange comprend :

- un acide fort (A) présentant un $pK_a$ (S) supérieur ou égal à celui de l'acide sulfurique et un $\Delta pK_a$ (S) par rapport à l'acide sulfurique inférieur ou égal à 4 et supérieur ou égal à 0
- et un autre acide (B) choisi parmi les superacides.

L'acide (A) présente un $pK_a$ (S) supérieur ou égal à celui de l'acide sulfurique : (S) représentant le solvant organique qui est le nitrobenzène.

L'acide (B) est un superacide défini comme présentant un $pK_a$ (S) inférieur à celui de l'acide sulfurique.

Le $pK_a$ (S) est défini comme la constante de dissociation ionique du couple acide/base dans un solvant (S).

On définit le $pK_a$ des acides par référence à une mesure de potentiométrie effectuée dans un solvant qui est le nitrobenzène (S) et dont le protocole de mesure est exposé avant les exemples de WO 2010/115784.

Les acides intervenant dans ledit mélange sont définis par une différence de $pK_a$, $\Delta$ $pK_a$ qui correspond pour un même solvant, à la différence entre le $pK_a$ de l'acide choisi et le $pK_a$ de l'acide sulfurique.

L'acide (A) mis en oeuvre présente un $\Delta pK_a$ (S) par rapport à l'acide sulfurique inférieur ou égal à 4 et supérieur ou égal à 0.

Encore plus préférentiellement, l'acide (A) a un $\Delta pK_a$ (S) par rapport à l'acide sulfurique inférieur ou égal à 3 et supérieur ou égal à 0.

Comme exemples d'acides (A), on peut citer notamment l'acide sulfurique, les acides sulfoniques aliphatiques ou aromatiques tels que par exemple, l'acide méthanesulfonique, l'acide éthanesulfonique, l'acide benzènesulfonique, les acides toluènesulfoniques, les acides naphtalènesulfoniques.

Une autre classe d'acides (A) sont les acides hydroxybenzènesulfoniques, les acides hydroxybenzoïques sulfonés ; les acides hydroxybenzènedisulfoniques, les acides dihydroxybenzène-disulfoniques les acides hydroxytoluènesulfoniques, les acides hydroxynaphtalènesulfoniques et les acides hydroxynaphtalènedisulfoniques et leurs mélanges.

Parmi les acides précités, les acides préférés sont : l'acide 4-hydroxybenzènesulfonique, l'acide 2-hydroxybenzènesulfonique, l'acide 5-sulfosalicylique, les acides sulfoniques résultant de la sulfonation de l'hydroquinone (1,4-dihydroxybenzène), du pyrocatéchol (1,2-dihydroxybenzène), et de la résorcine (1,3-dihydroxybenzène) ; l'acide 5,6-dihydroxy-1,3-benzènedisulfonique, l'acide 4,6-dihydroxy-1,3-benzènedisulfonique, l'acide 2,5-dihydroxy-1,4-benzènedisulfonique.

Comme autres exemples d'acides, on peut citer notamment les acides perhalogénoacétiques tels que l'acide trichloroacétique, l'acide trifluoroacétique.

Pour ce qui est du deuxième composant (B) du mélange d'acides, il s'agit d'un superacide c'est-à-dire un acide présentant un $pK_a$ (S) inférieur à celui de l'acide sulfurique et qui présente donc un $\Delta pK_a$ négatif.

La borne inférieure n'est pas critique mais généralement le $\Delta pK_a$ dans le nitrobenzène est supérieur ou égal à - 12.

Les superacides choisis préférentiellement présentent un $\Delta pK_a$ inférieur ou égal à - 0,1 et de préférence supérieur ou égal à - 8.

On peut citer comme exemples de superacides (B), l'acide perchlorique, les acides halogénosulfoniques tels que l'acide fluorosulfonique, l'acide chlorosulfonique ; les acides perhalogénoalcanesulfoniques, de préférence l'acide trifluorométhanesulfonique.

Egalement comme superacides (B), on peut mentionner entre autres, l'acide trifluorométhanesulfinique ; l'acide bis-trifluorométhanesulfonimide.

Comme couples d'acides (A) et (B) choisis préférentiellement, on peut mentionner l'acide perchlorique et l'acide sulfurique ; l'acide perchlorique et l'acide 4-hydroxybenzènesulfonique ; l'acide trifluorométhanesulfonique et l'acide 4-hydroxybenzènesulfonique ; l'acide bis-trifluorométhanesulfonimide et l'acide 4-hydroxybenzènesulfonique.

La proportion dans le mélange des différents acides peut varier largement. Ainsi, on peut mettre en oeuvre des mélanges comprenant :

- de 60 % à 95 %, de préférence de 80 % à 95 % en moles d'un acide (A),
- de 5 % à 40 % de préférence de 5 % à 20 % en moles d'un acide (B).

Chaque pourcentage d'acide exprime le rapport (exprimé en %) entre le nombre de moles de l'acide considéré et le nombre de moles de la somme des deux acides (A) et (B).

Les acides mis en oeuvre dans le mélange sont disponibles dans le commerce sous forme solide, liquide ou en solution aqueuse dont la concentration peut varier entre 5 et 95 % en poids, de préférence entre 50 et 70 % en poids.

L'acide protonique fort ou le mélange d'acides est mis en oeuvre dans le procédé de l'invention, en une quantité exprimée par le rapport du nombre d'équivalents de protons au nombre de moles de substrat phénolique qui varie avantageusement entre 0,002 et 0,15 %. Ainsi, ledit rapport molaire est choisi de préférence entre 0,01 % et 0,07 %.

Selon une autre variante du procédé de l'invention, on conduit l'hydroxylation du substrat phénolique, en présence d'un co-catalyseur qui est un composé cétonique et plus particulièrement ceux répondant à la formule (II) :

$$R_a\text{-CO-X-}R_b \qquad \text{(II)}$$

dans ladite formule (II) :

- $R_a$ et $R_b$, identiques ou différents, représentent des groupes hydrocarbonés ayant de 1 à 30 atomes de carbone ou forment ensemble un groupe divalent, éventuellement substitués par un ou plusieurs atomes d'halogène ou groupes fonctionnels stables dans les conditions de la réaction,
- X représente un lien valentiel, un groupe -CO-, un groupe -CHOH ou un groupe $-(R)_n-$ : R représentant un groupe alkylène ayant de préférence de 1 à 4 atomes de carbone et n est un nombre entier choisi entre 1 et 16.

Dans la formule (II), $R_a$ et $R_b$ représentent plus particulièrement :

- des groupes alkyle linéaires ou ramifiés,
- des groupes alcényle linéaires ou ramifiés,
- des groupes cycloalkyle ou cycloalcényle comportant de 4 à 6 atomes de carbone,
- des groupes aryle mono- ou polycycliques ; dans ce dernier cas, les cycles formant entre eux un système ortho- ou ortho- et péricondensé ou étant liés entre eux par un lien valentiel,
- des groupes arylalkyle ou arylacényle,
- $R_a$ et $R_b$ peuvent former ensemble un groupe alkylène ou alcénylène comportant de 3 à 5 atomes de carbone, éventuellement substitué par un groupe alkyle à faible condensation en carbone ou par un groupe cycloalkyle ou cycloalcényle ayant 4 à 6 atomes de carbone ; 2 à 4 des atomes de carbone des groupes alkylène ou alcénylène pouvant faire partie d'un ou deux cycles benzéniques éventuellement substitués par 1 à 4 groupes hydroxyle et/ou alkyle et/ou alkoxy à faible condensation en carbone.

Dans l'exposé qui suit de l'invention, on entend par « groupe alkyle de faible condensation en carbone », un groupe alkyle linéaire ou ramifié ayant généralement de 1 à 4 atomes de carbone.

Les groupes hydrocarbonés précités peuvent être substitués par 1 ou plusieurs, de préférence, 1 à 4, groupes alkyle de faible condensation en carbone ou groupes fonctionnels tels que les groupes hydroxyle, alkoxy à faible condensation en carbone, hydroxycarbonyle, alkyloxycarbonyle comportant de 1 à 4 atomes de carbone dans le groupe alkyle, un groupe nitrile, sulfonique, nitro ou par un ou plusieurs atomes d'halogènes, et notamment de chlore et de brome.

De préférence, $R_a$ et $R_b$ représentent plus particulièrement :

- des groupes alkyle linéaires ou ramifiés ayant de 1 à 10 atomes de carbone,
- des groupes alcényle linéaires ou ramifiés ayant de 2 à 10 atomes de carbone,
- des groupes cycloalkyle ou cycloalcényle comportant de 4 à 6 atomes de carbone,
- des groupes phényle éventuellement substitués par 1 à 4 groupes alkyle et/ou hydroxyle et/ou alkoxy,
- des groupes phénylalkyle ou phénylacényle comportant 1 (ou 2) à 10 atomes de carbone dans la partie aliphatique, et plus particulièrement encore de 1 (ou 2) à 5 atomes de carbone dans la partie aliphatique,
- $R_a$ et $R_b$ peuvent former ensemble un groupe alkylène ou alcénylène comportant de 3 à 5 atomes de carbone, éventuellement substitué par 1 à 4 groupes alkyle à faible condensation en carbone.

Ainsi, on fait appel, tout particulièrement, aux composés cétoniques de type dialkylcétone répondant à la formule (II) dans laquelle $R_a$ et $R_b$ représentent un groupe alkyle linéaire ou ramifié ayant de 1 à 8 atomes de carbone.

Parmi tous les composés cétoniques répondant à la formule (II), on choisit préférentiellement ceux qui répondent à la formule (II) dans laquelle $R_a$ et $R_b$ représentent un groupe phényle éventuellement substitué.

Lesdits composés cétoniques peuvent être représentés par la formule (IIa) suivante :

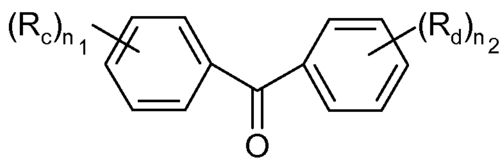

(IIa)

dans ladite formule (IIa) :

- $R_c$ et $R_d$, identiques ou différents représentent un atome d'hydrogène ou un substituant, de préférence un groupe électro-donneur,
- $n_1$, $n_2$, identiques ou différents est un nombre égal à 0, 1, 2 ou 3,
- éventuellement les deux atomes de carbone situés en position $\alpha$ par rapport aux deux atomes de carbone portant

le groupe -CO peuvent être liés ensemble par un lien valentiel ou par un groupe $-CH_2-$ formant ainsi un cycle cétonique qui peut être saturé mais aussi insaturé.

Le substituant est choisi de telle sorte qu'il ne réagisse pas dans les conditions d'acidité de l'invention. Il s'agit préférentiellement d'un groupe électro-donneur.

On entend par "groupe électro-donneur", un groupe tel que défini par H.C. BROWN dans l'ouvrage de Jerry MARCH - Advanced Organic Chemistry, chapitre 9, pages 243 et 244 (1985).

Des exemples de substituants convenant bien à l'invention sont les suivants :

- les groupes alkyle linéaires ou ramifiés ayant de 1 à 4 atomes de carbone,
- le groupe phényle,
- les groupes alkoxy comprenant une chaîne alkyle linéaire ou ramifié ayant de 1 à 4 atomes de carbone ou un groupe phénoxy,
- le groupe hydroxyle,
- l'atome de fluor.

Comme exemples de composés cétoniques particulièrement adaptés à l'invention, on peut citer tout particulièrement les composés cétoniques répondant à la formule générale (IIa) dans laquelle $R_c$ et $R_d$, identiques ou différents représentent un atome d'hydrogène ou un substituant tel que précité, de préférence en position 4,4' et $n_1$, $n_2$, identiques ou différents sont égaux à 0 ou 1.

On fait appel préférentiellement aux composés cétoniques répondant à la formule (IIa) dans laquelle $R_c$ et $R_d$, identiques ou différents représentent un atome d'hydrogène ; un groupe méthyle, éthyle, tert-butyle, phényle ; un groupe méthoxy ou éthoxy ; un groupe hydroxyle, de préférence en position 3,3' ou 4,4'.

Comme exemples spécifiques de cétones qui peuvent être utilisées dans le procédé de l'invention, on peut citer plus particulièrement :

- la benzophénone
- la 2-méthylbenzophénone
- la 2,4-diméthylbenzophénone
- la 4,4'-diméthylbenzophénone
- la 2,2'-diméthyl benzophénone
- la 4,4'-diméthoxybenzophénone
- la 4-hydroxybenzophénone
- la 4,4'-dihydroxybenzophénone
- le 4-benzoylbiphényle

La quantité de composé cétonique mise en jeu exprimée par le rapport entre le nombre de moles de composé cétonique et le nombre de moles de composé phénolique peut varier entre 0,01 % et 20 % et de préférence entre 0,1 % et 2 %.

Conformément au procédé de l'invention, on fait réagir un phénol ou un éther de phénol avec le peroxyde d'hydrogène, en présence d'un acide protonique fort et éventuellement d'une cétone.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène ayant une concentration en $H_2O_2$ d'au moins 20 % en poids et, de préférence, comprise entre 20 % et 90 % en poids.

On choisit avantageusement une solution aqueuse de peroxyde d'hydrogène ayant une concentration pondérale en $H_2O_2$ allant de 30 % à 90 %, de préférence de 30 % à 70 %, de manière plus préférée de 40% à 70% et de manière encore plus préférée de 45% à 70%.

La quantité de peroxyde d'hydrogène peut aller jusqu'à 0,5 mole de $H_2O_2$ pour 1 mole de substrat de formule (I).

Il est cependant préférable pour obtenir un rendement industriellement acceptable d'utiliser un rapport molaire peroxyde d'hydrogène/ substrat phénolique de 0,01 à 0,3 et, de préférence, de 0,03 à 0,10. Le substrat phénolique assure également avantageusement le rôle de solvant de manière à éviter l'emploi d'autres solvants, par exemple l'eau.

La quantité d'eau influençant la vitesse de la réaction, il est préférable de minimiser sa présence : l'eau pouvant être apportée dans le milieu réactionnel notamment par les réactifs mis en oeuvre.

Il convient de choisir préférentiellement une teneur initiale du milieu en eau inférieure à 20 % en poids et, de

préférence, inférieure à 10 % en poids.

Les teneurs pondérales en eau indiquées sont exprimées par rapport au mélange substrat de formule (I) - peroxyde d'hydrogène - eau.

Cette eau initiale correspond à l'eau introduite avec les réactifs et notamment avec le peroxyde d'hydrogène.

Une variante du procédé de l'invention consiste à ajouter un agent complexant des ions métalliques présents dans le milieu car ceux-ci sont préjudiciables au bon déroulement du procédé de l'invention, notamment dans le cas des phénols où les rendements en produits d'hydroxylation sont faibles. Par conséquent, il est préférable d'inhiber l'action des ions métalliques.

Les ions métalliques néfastes au déroulement de l'hydroxylation sont des ions de métaux de transition et plus particulièrement, les ions fer, nickel, cuivre, chrome, cobalt, manganèse et vanadium.

Les ions métalliques sont apportés par les réactifs et notamment les substrats de départ et l'appareillage utilisé. Pour inhiber l'action de ces ions métalliques, il suffit de conduire la réaction en présence d'un ou plusieurs agents complexants stables vis-à-vis du peroxyde d'hydrogène et donnant des complexes ne pouvant être décomposés par les acides forts présents et dans lesquels le métal ne peut plus exercer d'activité chimique.

A titre d'exemples non limitatifs d'agents complexants, on peut faire appel, notamment, aux divers acides phosphoriques tels que, par exemple, l'acide orthophosphorique, l'acide métaphosphorique, l'acide pyrophosphorique, les acides polyphosphoriques, les acides phosphoniques tels que l'acide (1-hydroxyéthylidène)diphosphonique, l'acide phosphonique, l'acide éthylphosphonique, l'acide phénylphosphonique.

On peut également mettre en oeuvre les esters des acides précités et l'on peut mentionner, plus particulièrement, les ortho phosphates de mono- ou di alkyle, de mono- ou dicycloalkyle, de mono- ou dialkylaryle, par exemple, le phosphate d'éthyle ou de diéthyle, le phosphate d'hexyle, le phosphate de cyclohexyle, le phosphate de benzyle.

La quantité d'agent complexant dépend de la teneur du milieu réactionnel en ions métalliques.

Il n'y a évidemment pas de limite supérieure, la quantité d'agents complexants présents pouvant être largement en excès par rapport à celle nécessaire pour complexer les ions métalliques. Généralement, une quantité représentant de 0,01 % et 2 %, de préférence de 0,01 à 0,3 % en poids du milieu réactionnel convient bien.

Conformément au procédé de l'invention, on conduit successivement le mélange des réactifs et la réaction d'hydroxylation.

Selon une première étape du procédé de l'invention, on met en contact le substrat phénolique et la solution de peroxyde d'hydrogène : le catalyseur acide étant introduit à l'étape du mélange et/ou en début de réaction d'hydroxylation.

Selon une première variante du procédé de l'invention, on additionne un agent complexant pour stabiliser le peroxyde d'hydrogène.

Selon une autre variante du procédé de l'invention, on met en oeuvre également un co-catalyseur.

D'un point de vue pratique, un mode préféré de mise en oeuvre des réactifs, consiste à introduire par voie séparée le composé phénolique éventuellement additionné d'un agent complexant, éventuellement tout ou partie du catalyseur acide et la solution de peroxyde d'hydrogène. Le co-catalyseur est aussi introduit par voie séparée.

L'opération de mélange est conduite à une température suffisante pour que le phénol ou l'éther de phénol de départ reste liquide. Ladite température est choisie en fonction de la température de fusion du substrat phénolique. S'agissant du phénol, par exemple, l'opération de mélange est réalisée à une température généralement supérieure à 41 voire 42°C.

L'opération de mélange est conduite de telle sorte que la réaction ne débute pas ou débute peu au cours de cette étape.

Ainsi, le taux de transformation du peroxyde d'hydrogène est inférieur à 25 % en mol, de préférence compris entre 0,5 % et 25 % et encore plus préférentiellement entre 0,5 % et 15 % en mol.

Pour ce faire, la température de cette opération de mélange est choisie avantageusement au plus égale à 85°C, de préférence comprise entre 45°C et 60°C.

La température lors de l'opération de mélange est choisie différemment selon que le catalyseur est introduit lors de cette étape de mélange ou en début d'hydroxylation.

En effet, lorsqu'il est introduit tout ou en partie, lors du mélange, il est souhaitable afin de minimiser le taux de transformation du peroxyde d'hydrogène, de choisir une température plus basse et se situant dans l'intervalle défini préférentiellement entre 45°C et 60°C.

Si le catalyseur est introduit dans sa totalité en début de réaction d'hydroxylation, la température de l'opération de mélange peut avoir lieu à une température plus élevée pouvant atteindre 85°C.

Le mélange peut être effectué sous pression atmosphérique mais des pressions supérieures peuvent être également envisagées. Par exemple, des pressions entre 1 et 200 bar absolus peuvent convenir.

On peut conduire cette étape, sous une atmosphère inerte par exemple sous azote ou bien encore sous argon, l'azote étant préféré notamment compte tenu de son coût réduit.

Le temps de séjour et la température du milieu réactionnel dans le dispositif de mélange doivent être en

adéquation avec le taux de transformation du peroxyde d'hydrogène choisi dans ledit dispositif.

Après cette opération de mélange des réactifs, on conduit la réaction d'hydroxylation.

Il est à noter qu'au cours de cette réaction, le milieu réactionnel est préférentiellement un système monophasique comprenant une phase liquide mais l'invention n'exclut pas des systèmes biphasiques gaz-liquide.

Selon une caractéristique du procédé de l'invention, la réaction d'hydroxylation est conduite dans des conditions adiabatiques.

La chaleur dégagée par la réaction suffit à elle seule à l'avancement de la réaction sans qu'il soit nécessaire de faire une régulation externe de température.

Ainsi, la réaction est conduite dans un réacteur qui présente la caractéristique d'être isolé thermiquement de l'extérieur de manière à ce qu'elle soit réalisée dans des conditions adiabatiques. Compte tenu de l'exothermie de la réaction, la température augmente naturellement dans le réacteur.

Il peut s'agir d'un réacteur en matériau isolant tel que différents polymères PVDF (polyfluorure de vinylidène), PVC (polychlorure de vinyle, PTFE (polytétrafluoroéthylène) chargé ou non par exemple par du carbone ou des fibres de verre. Un réacteur en verre ou en acier vitrifié peut également convenir.

Un autre moyen d'isoler le réacteur notamment dans le cas de réacteurs à ossature métallique est de le calorifuger afin d'éviter tout échange thermique avec l'extérieur.

Le calorifugeage peut être assuré par enveloppement du réacteur par un calorifuge tel que laine de verre, laine de roche, mousse synthétique isolante, notamment mousse de polyuréthane, calorifuge éventuellement recouvert par un revêtement métallique, par exemple, en acier ordinaire ou inoxydable.

La réaction peut être conduite sous pression atmosphérique mais des pressions supérieures peuvent être également envisagées comme précédemment mentionné.

On peut conduire cette étape, sous une atmosphère inerte, de préférence sous atmosphère d'azote.

En fin de réaction, le produit d'hydroxylation peut être soit séparé du substrat non transformé, et le cas échéant les catalyseurs et co-catalyseurs par les moyens usuels, notamment, par distillation et/ou extraction liquide/liquide et sont renvoyés dans la zone réactionnelle ou bien soit engagé directement dans une étape le faisant intervenir comme substrat de départ.

Conformément à une variante d'exécution de l'invention, on conduit le mélange des réactifs et la réaction d'hydroxylation simultanément.

Pour ce faire, on préchauffe au moins le composé phénolique à une température supérieure à 70°C, de préférence comprise entre 70°C et 85°C avant de conduire la réaction dans des conditions adiabatiques.

Le composé phénolique et la solution de peroxyde d'hydrogène peuvent être mis en oeuvre par voie séparée.

Le catalyseur acide peut être introduit par voie séparée tout ou partie, en début de réaction.

Il est également possible de faire des prémélanges de réactifs, substrat phénolique et solution de peroxyde d'hydrogène : le catalyseur étant alors introduit par voie séparée.

Dans le cas de la présence d'un agent complexant, celui-ci peut être introduit par exemple dans le substrat phénolique.

Dans cette variante, on préchauffe au moins le composé phénolique mais il est également possible ou non de préchauffer les autres réactifs tels que solution de peroxyde d'hydrogène et catalyseur acide.

La réaction est conduite comme décrite ci-dessus dans des conditions adiabatiques.

Selon un mode préféré de réalisation de l'invention, on effectue le mélange des réactifs dans un dispositif de mélange pourvu d'une agitation et de moyens de chauffage et l'on réalise ensuite l'hydroxylation dans des conditions adiabatiques dans un réacteur à écoulement piston.

Selon un autre mode de réalisation de l'invention, on conduit l'étape de mélange des réactifs et l'étape d'hydroxylation dans des conditions adiabatiques dans un réacteur à écoulement piston. Dans ce cas, les fluides (au moins le substrat phénolique) doit être préchauffé, afin d'amorcer la réaction

Selon un autre mode de réalisation de l'invention, on réalise la deuxième étape du procédé à savoir l'hydroxylation dans une succession d'au moins deux réacteurs à écoulement piston maintenus dans des conditions adiabatiques et séparés par un échangeur thermique.

En effet pour limiter les phénomènes de suroxydation conduisant à la formation des lourds, il peut être utile de conduire l'hydroxylation en plusieurs temps et donc plusieurs étages peuvent être envisagés.

Ainsi, on débute la réaction d'hydroxylation en mode adiabatique et l'on soumet le mélange réactionnel obtenu à un refroidissement par passage dans un échangeur thermique afin d'abaisser sa température de 5°C à 90°C et de préférence de 10°C à 40°C.

On poursuit l'hydroxylation dans une deuxième section en envoyant ledit mélange dans un deuxième réacteur adiabatique et ainsi de suite.

Enfin, selon un autre mode de réalisation de l'invention, on effectue le mélange des réactifs dans un dispositif de mélange pourvu d'une agitation et de moyens de chauffage et l'on réalise la deuxième étape d'hydroxylation dans un faisceau de réacteurs à écoulement piston montés en parallèle.

D'une manière avantageuse, intervient pour la mise en oeuvre du procédé de l'invention, au moins un réacteur à écoulement piston.

Par « écoulement piston », on définit un écoulement unidirectionnel dans lequel dans un plan perpendiculaire à l'écoulement, tous les filets fluides se déplacent avec une vitesse uniforme. Dans un tel écoulement, le mélange radial est parfait alors qu'il n'y a pas de mélange axial. En pratique, ces conditions sont considérées comme remplies lorsque l'écoulement est turbulent.

On estime qu'un écoulement est turbulent lorsque le nombre de Reynolds est supérieur ou égal à 5000, et préférentiellement lorsqu'il est supérieur à 10000. Lorsque l'écoulement n'est pas turbulent, le mélange radial n'est pas parfait et il peut y avoir rétromélange axial. Dans ce cas, pour un nombre de Reynolds inférieur à environ 5000 et plus particulièrement inférieur à 2000, on garnit le réacteur tubulaire ou le réacteur colonne avec des internes et/ou on le structure.

On rappelle que la définition du nombre de Reynolds est :

$$Re = \frac{\rho \, . \, v \, . \, d}{\mu}$$

dans laquelle :

- $\rho$ est la masse volumique du fluide en kg/m$^3$;
- v est la vitesse d'écoulement en m/s ;
- d est le diamètre du réacteur en m
- $\mu$ est la viscosité dynamique en Pa.s

Généralement, Re est compris entre 1 et 1 000 000.

Selon des modes de réalisation préférés, le réacteur à écoulement piston dans lequel est conduite la réaction d'hydroxylation est un réacteur tubulaire ou un réacteur colonne.

Dans l'exposé qui suit la présente invention, on entend par « réacteur tubulaire », un réacteur en forme de tube et par « réacteur colonne » un réacteur vertical de section circulaire.

L'invention sera mieux comprise à la lecture de la description qui va suivre, donnée uniquement à titre d'exemple, et faite en se référant aux dessins 1 à 7 annexés.

Un premier mode de réalisation pratique de l'invention est illustré par le dessin annexé sous forme de la figure 1.

La figure 1 est une vue schématique d'un appareillage adapté à la mise en oeuvre de l'invention et qui comprend deux ensembles.

Le premier ensemble comprend un réacteur agité à double enveloppe (1) équipé de moyens d'introduction des réactifs.

Le deuxième ensemble comprend un réacteur (2) à écoulement piston.

A la sortie du réacteur agité (1), on obtient un mélange réactionnel qui est introduit dans le réacteur à écoulement piston (2).

La figure 2 est une vue schématique d'un appareillage qui comprend comme sur la figure 1, deux ensembles mais qui se différencie seulement par le fait que le réacteur agité à double enveloppe est remplacé par un mélangeur statique à double enveloppe (13) chauffé par un fluide caloporteur circulant dans une double enveloppe qui entre en (30) et qui sort en (31).

Un autre mode de réalisation de l'invention est représenté par le dessin annexé sous forme de la figure 3.

L'appareillage réside dans un réacteur à écoulement piston (2) équipé des moyens d'introduction des réactifs.

Les réactifs éventuellement préchauffés par passage dans un échangeur thermique sont introduits dans le réacteur à écoulement piston (2).

La figure 4 est une vue schématique d'un appareillage adapté pour la mise en oeuvre d'une variante du procédé de l'invention selon laquelle l'installation est configurée de telle sorte que la température de la réaction soit étagée.

L'appareillage utilisé est constitué de plusieurs ensembles.

Le premier ensemble comprend le réacteur (1) équipé de moyens d'introduction des réactifs et de mélange.

Le deuxième ensemble comprend au moins deux réacteurs (2) et (14) à écoulement piston séparés par un échangeur thermique.

A la sortie du réacteur agité (1), on obtient un mélange réactionnel qui est introduit dans le réacteur adiabatique à écoulement piston (2) et qui traverse ensuite l'échangeur thermique dans lequel il est refroidi par un fluide caloporteur (15) pour être ensuite introduit dans le réacteur à écoulement piston (14).

En sortie du réacteur (19), on obtient le produit hydroxylé.

Selon des modes de réalisation préférés, le réacteur dans lequel est conduite la réaction d'hydroxylation est un réacteur tubulaire ou un réacteur colonne.

La figure 5 est une vue schématique d'un appareillage qui comprend comme sur la figure 1, deux ensembles mais qui se différencie seulement par le fait que le réacteur à écoulement piston est remplacé par un faisceau de réacteurs à écoulement piston montés en parallèle.

Les figures 6 et 7 illustrent des types de réacteurs à écoulement piston susceptibles d'être utilisés dans le procédé de l'invention.

La figure 6 est une représentation schématique d'un réacteur tubulaire formé de tubes concentriques.

La figure 7 est une représentation schématique d'un réacteur en forme de colonne.

Un exposé plus détaillé des moyens de l'invention est donné en se référant aux figures 1 à 7 qui schématisent les étapes du procédé de l'invention, sans pour autant lier la portée de l'invention, à celui-ci.

Selon différents modes de réalisation de l'invention, on peut effectuer les étapes de mélange et d'hydroxylation :

- successivement c'est-à-dire que le mélange réactionnel effectué dans une première capacité équipé de moyens de chauffage, passe ensuite dans le réacteur à écoulement piston,
- ou simultanément à savoir que les réactifs sont mélangés et tout de suite soumis à l'étape d'hydroxylation par introduction dans le réacteur à écoulement piston : l'un ou plusieurs des réactifs étant préchauffés par passage dans un échangeur thermique avant leur introduction dans ledit réacteur.

Les figures 1 et 2 illustrent le procédé de l'invention dans lequel le mélange des réactifs est effectué dans un dispositif de mélange avant son introduction dans le réacteur à écoulement piston.

Le mélange des réactifs est susceptible d'être agité et chauffé dans ce dispositif de mélange.

Le réacteur agité à double enveloppe représenté sur la figure (1) et le mélangeur statique à double enveloppe (13) sont des exemples de dispositifs de mélange mais d'autres types peuvent être utilisés comme mentionné ci-dessous.

Une première classe de dispositifs de mélange concerne les réacteurs agités mécaniquement. Le réacteur est généralement de forme cylindrique verticale avec fond plat ou elliptique.

Ce réacteur est équipé de moyens d'introduction des réactifs, de moyens de chauffage, d'un système d'agitation et dans sa partie inférieure ou supérieure, d'un système de soutirage du mélange réactionnel. Le réacteur est également muni de dispositif de mesure de température et de pression.

Le mélange est conduit dans un réacteur présentant de bonnes performances en termes de transfert de matière et de transfert thermique.

Le système d'agitation non représenté sur la figure 1 peut être un agitateur rotatif.

Comme exemples d'agitateurs, on peut mentionner, entre autres, la turbine à pales droites ou inclinées ou l'hélice marine ou tout mobile "hydrofoil".

Une deuxième classe de dispositifs de mélange a trait aux boucles externes. Le mélange du milieu réactionnel est alors fait par circulation en boucle d'une fraction ou totalité du contenu d'un réacteur agité mécaniquement ou non, à l'aide d'une pompe sur boucle externe.

Une troisième classe de dispositifs de mélange illustré par la figure 2 regroupe les mélangeurs sans pièce en rotation, dits mélangeurs dynamiques, d'une part, et statiques, d'autre part.

Dans la famille des mélangeurs dynamiques, on peut citer les mélangeurs à jets tangentiels, les mélangeurs à jets d'impact ou encore les éjecteurs.

Pour les mélangeurs dits statiques, on peut lister différents internes, tels que les mélangeurs statiques (Sulzer SMX, Kenics, etc...), un lit de billes ou particules en vrac, des mousses métalliques ou céramiques, etc...

L'ensemble de ces mélangeurs forcent les filets de fluides alimentés à échanger la matière entre eux en se divisant en sub-filets ou en créant des structures à petite échelle. Ces structures augmentent la surface de contact entre les flux de réactifs.

La surface d'échange pour assurer le transfert thermique peut être augmentée par des serpentins ou des plaques présentes à l'intérieur du réacteur ou par l'intermédiaire d'un fluide caloporteur circulant dans une double enveloppe.

Comme fluides caloporteurs, on peut mentionner entre autres, l'eau, la vapeur d'eau ou un solvant organique approprié comme par exemple un éther aromatique comme l'oxyde de biphényle et/ou l'oxyde de benzyle, une huile silicone, une huile paraffinique et/ou naphténique, résidus de distillation du pétrole, etc...

D'un point de vue pratique, on introduit dans le réacteur agité à double enveloppe (1) selon la figure 1 ou dans le mélangeur statique (13) selon la figure 2, le substrat phénolique (3) et une solution de peroxyde d'hydrogène (4).

Dans le cas de la présence d'un agent complexant, celui-ci peut être introduit par exemple dans le substrat phénolique.

Un dispositif d'alimentation du co-catalyseur peut être également envisagé.

Le catalyseur est introduit en (5) ou éventuellement en tête (6) du réacteur à écoulement piston.

L'introduction du catalyseur peut être également en (5) et (6).

Les différents réactifs sont introduits progressivement, de préférence en continu : leur débit d'introduction étant régulé par l'intermédiaire de pompe.

Le mélange des réactifs est obtenu comme mentionné précédemment et la figure 1 illustre la réalisation du mélange en établissant une boucle de recirculation comme illustré sur la figure 1.

Une partie du mélange réactionnel est prélevé en pied de réacteur en (7) et est ensuite introduit dans le réacteur en (8) : la circulation forcée du mélange étant assurée par une pompe non représentée sur le schéma.

Selon la figure 2, les réactifs sont mélangés grâce à la structure même du mélangeur statique.

Le chauffage est assuré par circulation d'un fluide caloporteur dans une double enveloppe.

Comme mentionné précédemment, le mélange est effectué dans des conditions telles que la réaction soit minimisée, et que le taux de transformation du peroxyde d'hydrogène soit inférieur à 25 % en mol.

Ainsi, la température de cette opération de mélange est choisie avantageusement au plus égale à 85°C, de préférence comprise entre 45°C et 60°C.

La réaction est conduite avantageusement sous pression atmosphérique mais des pressions supérieures peuvent être également envisagées. Par exemple, des pressions entre 1 et 200 bar absolus peuvent convenir.

On peut conduire avantageusement cette étape, sous une atmosphère inerte par exemple sous azote ou bien encore sous argon, l'azote étant préféré notamment compte tenu de son coût réduit.

Le temps de séjour et la température du milieu réactionnel dans le dispositif de mélange doivent être en adéquation avec le taux de transformation du peroxyde d'hydrogène choisi dans ledit dispositif.

Le mélange réactionnel passe ensuite du réacteur (1) au réacteur (2) selon la figure 1 ou du mélangeur (13) au réacteur (2) selon la figure 2, par écoulement gravitaire ou par circulation forcée par exemple à l'aide d'une pompe, le plus souvent une pompe centrifuge.

Conformément au procédé de l'invention, on effectue une deuxième étape relative à la réaction d'hydroxylation conduite dans un réacteur à écoulement piston (2).

Le réacteur est constitué d'un tube (9) à l'intérieur duquel circulent le mélange réactionnel qui entre en (10) et les produits de la réaction qui sortent en (11).

Le réacteur présente la caractéristique d'être calorifugé. Il est enveloppé d'un revêtement calorifuge (12).

Le plus souvent, le réacteur présentera un rapport longueur / diamètre supérieur à 3. Il peut s'agir notamment d'un réacteur tubulaire présentant un rapport longueur / diamètre compris entre 4 et 30 et en particulier compris entre 5 et 10.

Avantageusement, le réacteur tubulaire est formé de manière à avoir un faible encombrement et permet d'augmenter le caractère piston, par exemple lorsqu'il est replié.

Le matériau du réacteur n'est pas particulièrement limité. Il sera choisi de manière à être inerte dans les conditions réactionnelles. calorifugé ou en polymère qui présente tant l'avantage d'éviter la corrosion que de conserver la chaleur de la réaction au sein du milieu réactionnel.

Les réacteurs tubulaires sont généralement disposés à l'horizontale.

Toutefois, afin de s'adapter aux contraintes spatiales, il peut également être envisagé de prévoir un réacteur disposé à la verticale ou encore incliné.

Avantageusement, un ou plusieurs plateaux perforés sont disposés à proximité de l'entrée des réactifs afin d'assurer une bonne homogénéité des fluides dans cette section du réacteur.

Avantageusement, le réacteur tubulaire est en forme de colonne. Il est équipé de conduits d'entrée des réactifs et de sortie du mélange réactionnel.

L'alimentation des réactifs dans le réacteur (1) est réalisée par des moyens classiques tels que par exemple une pompe et plus particulièrement une pompe centrifuge ou une pompe volumétrique.

En principe, il est préféré de travailler en phase liquide seulement.

Le réacteur tubulaire peut être équipé d'internes.

La présence dans le réacteur d'internes crée des turbulences qui assurent une homogénéité du mélange réactionnel sur toute la section du réacteur. Les internes permettent ainsi de maintenir le caractère d'écoulement piston, y compris pour un nombre de Reynolds inférieur à 5000.

Le matériau des internes importe peu, du moment qu'il est chimiquement inerte vis-à-vis du mélange réactionnel dans les conditions de la réaction. Généralement, il s'agit de matériaux tels que du verre, du métal, notamment de l'acier inoxydable, du carbone, du polymère ou encore de la céramique.

Différents types d'internes peuvent être envisagés. Il peut s'agir notamment :

- d'internes en vrac, qui consistent en de petits objets par exemple sous forme d'anneaux, de selles, de boules ou cylindres creux, dont est rempli tout ou partie du réacteur.
- des internes structurés : clavettes, mélangeurs statiques, chicanes.

De préférence, les internes sont disposés dans le réacteur à proximité de l'entrée des réactifs.

Dans le cas d'un réacteur disposé à la verticale, les internes sont disposés de préférence sur toute la hauteur du réacteur. Il y a alors lieu de prévoir un support approprié, par exemple sous forme de croisillons, afin de maintenir les internes en place.

Particulièrement préféré est un garnissage de type mélangeur statique, composé d'éléments de mélange comportant des lames de guidage arrangées selon des angles précis et disposées de manière complexe. Ce type de garnissage est par exemple commercialisé par la société SULZER sous la dénomination SMV et SMX.

Pour la description de ces internes, on peut se référer à l'article « Don't Be Baffled By Static Mixers » paru dans Chemical Engineering, May 2003.

D'un point de vue pratique, on choisit un tube linéaire sans internes replié sur lui-même et disposé horizontalement ou verticalement lorsque le nombre de Reynolds est supérieur à 2000, et préférentiellement lorsqu'il est supérieur à 5000.

Lorsque le nombre de Reynolds est inférieur à 5000, on peut mettre en oeuvre un réacteur sans internes et en le structurant. Par exemple, il est possible de replier le réacteur tubulaire sur lui-même sous la forme d'une hélice ou d'une succession de coudes - lignes droites ; cette structure peut éventuellement être équipée d'internes partiellement. Après chaque coude du tube, on introduit un tronçon d'internes d'une longueur équivalente par exemple de 3 à 6 fois le diamètre du tube, sur toute ou partie de la longueur droite du tube située entre deux coudes successifs.

Dans cette étape de procédé, la température n'est pas contrôlée puisque les conditions sont adiabatiques.

Compte tenu de l'exothermie de la réaction, on précise à titre indicatif que la température de la réaction à l'entrée du réacteur à écoulement piston est supérieure à 70°C, de préférence comprise entre 70°C et 85°C.

La réaction est conduite avantageusement sous pression atmosphérique mais également des pressions supérieures peuvent être envisagées comme précédemment.

De fait, à haute température, donc vers la sortie du réacteur, il se peut que le substrat phénolique se vaporise partiellement : il est alors moins disponible pour être converti. Pour opérer sous pression, le réacteur est équipé soit d'un déverseur à l'échelle laboratoire ou pilote, soit d'une vanne de régulation en aval du réacteur, afin de réguler la pression à la valeur souhaitée.

On peut conduire avantageusement cette étape, sous une atmosphère inerte, de préférence sous atmosphère d'azote.

En fin de réaction, le produit hydroxylé présent dans le mélange réactionnel est récupéré en (11).

La figure 3 illustre un mode de mise en oeuvre du procédé de l'invention selon lequel le mélange des différents réactifs est effectué à l'entrée du réacteur à écoulement piston.

Le substrat phénolique (3) et la solution de peroxyde d'hydrogène (4) peuvent être introduits dans le réacteur par voie séparée.

Le catalyseur acide peut être introduit par voie séparée à l'entrée du réacteur (5) et/ou plus à l'intérieur, en début de réacteur (6) comme le montre la figure 3.

Il est également possible de faire des prémélanges de réactifs, substrat phénolique et solution de peroxyde d'hydrogène : le catalyseur pouvant alors être introduit par voie séparée à l'entrée du réacteur (5) et/ou plus à l'intérieur en début de réacteur (6).

Dans le cas de la présence d'un agent complexant, celui-ci peut être introduit par exemple dans le substrat phénolique.

Le co-catalyseur peut être également ajouté par voie séparée.

Selon ce mode de réalisation, il y a lieu de préchauffer au moins le substrat phénolique avant son introduction dans le réacteur (2).

Il est également possible ou non de préchauffer les autres réactifs tels que solution de peroxyde d'hydrogène et catalyseur acide.

La température de préchauffage est choisie avantageusement supérieure à 70°C, de préférence comprise entre 70°C et 85°C.

Le préchauffage des réactifs est effectué par passage dans un échangeur thermique.

Les réactifs dont au moins le substrat phénolique est préchauffé, sont introduits dans le réacteur à écoulement piston ayant des caractéristiques telles que décrites pour la figure 1.

On récupère en sortie de réacteur (2), un mélange réactionnel (11) comprenant le produit hydroxylé.

La figure 4 illustre un mode de mise en oeuvre du procédé de l'invention selon lequel on effectue une première étape de mélange des réactifs dans le dispositif de mélange (1) puis on conduit l'étape d'hydroxylation de telle sorte que la température de la réaction soit étagée dans au moins deux réacteurs à écoulement piston travaillant dans des conditions adiabatiques.

Pour la première étape, d'une manière identique telle qu'illustrée par la figure (1), on introduit dans le dispositif de mélange (1), le substrat phénolique (3) et une solution de peroxyde d'hydrogène (4).

Dans le cas de la présence d'un agent complexant, celui-ci peut être introduit par exemple dans le substrat

phénolique.

Un dispositif d'alimentation du co-catalyseur peut être également envisagé.

Le catalyseur est introduit en (5) ou éventuellement en tête (6) du réacteur à écoulement piston.

En sortie du dispositif de mélange, le mélange de réactifs (10) est introduit dans une succession d'au moins deux réacteurs à écoulement piston séparés par un échangeur thermique.

Dans ce mode de réalisation, les réacteurs fonctionnent toujours en adiabatique mais avec différentes zones thermiques.

Chaque étage comprend un réacteur à écoulement piston et un échangeur thermique : l'installation se finissant par un réacteur à écoulement piston.

Le nombre d'étages peut varier par exemple de 2 à 100, de préférence de 2 à 10.

La température dans chaque étage est déterminée selon le taux de transformation du peroxyde d'hydrogène souhaité.

La figure 4 représente, uniquement à titre illustratif, un ensemble comprenant au moins deux réacteurs à écoulement piston (2) et (14) séparés par un échangeur thermique (15). Les différentes températures dans chaque étage sont assurées par la présence d'un échangeur thermique dont la fonction est de refroidir le mélange réactionnel à la sortie d'un premier réacteur avant son entrée dans le suivant afin de maximiser la conversion du substrat phénolique tout en minimisant sa suroxydation en lourds.

L'étagement de température et le temps de séjour de chaque zone sont établis en adéquation avec les performances réactionnelles (taux de transformation et rendements).

Cette détermination peut être faite conformément à l'ouvrage de J. Villermaux (« Génie de la réaction chimique ; conception et fonctionnement des réacteurs»; J. Villermaux ; Tec & Doc Lavoisier ; 1993) ou celui de 'O. Levenspiel (« . Chemical Reaction Engineering » ; 2nd edition ; Wiley Int. , 1972).

L'étagement de la température dans le procédé selon l'invention est donc réalisé grâce à la présence des échangeurs thermiques entre les réacteurs.

En se référant à la figure 4 qui illustre un mode de réalisation, le mélange réactionnel (10) traverse le réacteur à écoulement piston (2) et à sa sortie (11) est introduit en (16) dans un échangeur thermique (15).

Le mélange réactionnel refroidi ou condensé (17) en sortie d'échangeur thermique (17) est introduit en (18) dans le réacteur à écoulement piston (14) suivant.

Le mélange en sortie (19) comprend le produit hydroxylé.

Il est donc possible de prévoir d'ajouter une succession de réacteurs à écoulement piston et d'échangeurs thermiques.

La réaction est conduite avantageusement sous pression atmosphérique mais des pressions supérieures entre 1 et 200 bar absolus peuvent être également envisagées comme précédemment.

On peut conduire avantageusement cette étape, sous une atmosphère inerte.

La figure 5 illustre un mode de mise en oeuvre du procédé de l'invention selon lequel on effectue une première étape de mélange des réactifs dans le dispositif de mélange (1) puis l'on conduit l'étape d'hydroxylation dans un faisceau de réacteurs à écoulement piston montés en parallèle et travaillant dans des conditions adiabatiques.

Pour la première étape, d'une manière identique telle qu'illustrée par la figure (1), on introduit dans le dispositif de mélange (1), le substrat phénolique (3) et une solution de peroxyde d'hydrogène (4).

Dans le cas de la présence d'un agent complexant, celui-ci peut être introduit par exemple dans le substrat phénolique.

Un dispositif d'alimentation du co-catalyseur peut être également envisagé.

Le catalyseur est introduit en (5) ou éventuellement en tête (6) de chaque réacteur à écoulement piston.

En sortie du dispositif de mélange, le mélange de réactifs (10) est divisé selon la figure 5 en trois fractions : chaque fraction étant introduite dans un réacteur à écoulement piston (32), (33) et (34) fonctionnant en adiabatique.

Dans ce mode de réalisation, le nombre de réacteurs à écoulement piston est de 3 sur la figure 5 mais peut varier par exemple de 2 à 100.

On récupère en sortie de chaque réacteur, un mélange réactionnel comprenant le produit hydroxylé : les 3 flux sortant des réacteurs étant regroupés en (11).

Les figures annexées 6 et 7 illustrent le type d'appareillages susceptibles d'être utilisés comme réacteur à écoulement piston.

La figure 6 représente un réacteur tubulaire formé de tubes concentriques.

Ainsi, le réacteur est constitué d'un tube (20) à l'intérieur duquel circulent le mélange réactionnel qui entre en (21) et les produits de la réaction qui sortent en (22).

Le tube est calorifugé (23).

Le tube peut contenir des tronçons de garnissage après chaque coude (24).

La figure 7 représente un réacteur en forme de colonne (25) qui est calorifugé (26).

La colonne est munie d'internes (27).

Le mélange réactionnel est introduit en (27) et les produits ressortent en (28).

Comme mentionné précédemment, le procédé de l'invention permet d'obtenir un accroissement significatif de la productivité de l'appareillage.

Le procédé de l'invention peut être mis en oeuvre dans un réacteur à écoulement piston ou dans un appareillage enchaînant un dispositif de mélange dans lequel est fait le mélange des réactifs et d'un réacteur à écoulement piston dans lequel est conduite la réaction d'hydroxylation.

Un avantage du procédé de l'invention de conduire la réaction d'hydroxylation du substrat phénolique dans un réacteur à écoulement piston permet un gain de sélectivité comparé à un procédé où la réaction d'hydroxylation est conduite dans une cascade de réacteurs agités. En effet, une bonne sélectivité a été obtenue en raison d'une limitation des réactions subséquentes.

Un autre avantage du procédé de l'invention est que le taux de transformation du substrat phénolique est accru, par exemple allant de 5 % à 15 %, préférentiellement de 5 % à 10% de sorte que la quantité de substrat phénolique résiduaire, à recycler est moindre ce qui permet de baisser la consommation énergétique.

Selon le mode d'exécution de l'invention dans un appareillage comprenant un dispositif de mélange couplé à un réacteur à écoulement piston, le fait que le mélange des réactifs soit fait en amont du réacteur à écoulement piston dans un dispositif séparé dudit réacteur à écoulement piston permet d'accroître la sécurité du procédé et il est alors possible de faire appel à des solutions aqueuses de peroxyde d'hydrogène plus concentrées dont la concentration peut varier de 30 % à 90 % en poids et de préférence de 30 % à 70 %.

Par ailleurs, le fait de n'avoir qu'un seul dispositif de mélange couplé à un réacteur à écoulement piston présente l'avantage d'un faible encombrement ainsi qu'une économie de coûts de fonctionnement, d'énergie et d'investissement comparativement à une cascade de réacteurs parfaitement agités, équipés chacun de moyens d'introduction des réactifs, de soutirage des produits, ainsi que des dispositifs de mélange des réactifs et de contrôle des paramètres de procédé.

Un autre intérêt de l'invention est qu'il conduit à de bons rendements réactionnels.

L'invention sera expliquée plus en détail au moyen d'exemples qui illustrent l'invention sans toutefois la limiter.

Dans les exemples, les abréviations suivantes signifient :

Le taux de transformation ($TT_{H2O2}$) du peroxyde d'hydrogène correspond au rapport entre le nombre de moles de peroxyde d'hydrogène transformées et le nombre de moles de peroxyde d'hydrogène introduites.

Le taux de transformation ($TT_{phenol}$) du phénol correspond au rapport entre le nombre de moles de phénol transformées et le nombre de moles de phénol introduites.

Le rendement en diphénols ($RR_{diphénols}$) correspond au rapport entre le nombre de moles de diphénols formées (pyrocatéchol + hydroquinone) et le nombre de moles de peroxyde d'hydrogène introduites.

Le rendement en pyrocatéchol ($RR_{pyrocatéchol}$) correspond au rapport entre le nombre de moles de pyrocatéchol formées et le nombre de moles de peroxyde d'hydrogène introduites.

Le rendement en hydroquinone ($RR_{hydroquinone}$) correspond au rapport entre le nombre de moles d'hydroquinone formées et le nombre de moles de peroxyde d'hydrogène introduites.

La sélectivité en diphénols ($RT_{diphénols}$) correspond au rapport entre le nombre de moles de diphénols formées (pyrocatéchol + hydroquinone) et le nombre de moles de peroxyde d'hydrogène transformées.

Le ratio PC/HQ est défini par le rapport entre le nombre de moles de pyrocatéchol et le nombre de moles d'hydroquinone.

Exemple 1

L'exemple est réalisé dans un type d'appareillage tel qu'illustré par la figure 1.

Dans un réacteur de volume utile 150 mL muni d'une double enveloppe et équipé d'un système d'agitation type 4 pales inclinées, d'un réfrigérant ascendant, d'une arrivée d'azote et d'un système de régulation de la température, on introduit à l'aide de pompes à 50°C en continu :

- 883 g/h (9,38 mol/h) de phénol contenant l'acide pyrophosphorique à raison de 400 ppm massique par rapport au phénol,
- l'acide perchlorique à raison de 400 ppm molaires par rapport au phénol,
- 26,0 g/h de peroxyde d'hydrogène à 70 % en poids (soit 0,53 mol/h de peroxyde d'hydrogène).

Le temps de passage dans ce réacteur est de 10 minutes ; le milieu étant maintenu à 50°C.

Le milieu réactionnel de ce réacteur est introduit en continu à l'aide d'une pompe dans un échangeur dans lequel il est chauffé à 80°C puis dans un réacteur tubulaire garni de mélangeurs Sulzer SMX de 57 mL de volume global (longueur = 200 mm; diamètre = 19 mm); ce réacteur étant calorifugé et maintenu en pression par un clapet taré à 10 bar en sortie. Le temps de passage dans l'échangeur est de l'ordre de 20 secondes.

Après une durée de stabilisation (environ ½ h), on dose les diphénols formés par chromatographie liquide

haute performance et le peroxyde d'hydrogène par potentiométrie.

Les résultats obtenus à la sortie du réacteur tubulaire sont consignés dans le tableau (I).

Exemple 2

Dans un réacteur de volume utile 150 mL muni d'une double enveloppe et équipé d'un système d'agitation type 4 pales inclinées, d'un réfrigérant ascendant, d'une arrivée d'azote et d'un système de régulation de la température, on introduit à l'aide de pompes à 50°C en continu :

- 831 g/h (8,83 mol/h) de phénol contenant l'acide pyrophosphorique à raison de 400 ppm massique par rapport au phénol,
- l'acide perchlorique à raison de 400 ppm molaires par rapport au phénol,
- 34,3 g/h de peroxyde d'hydrogène à 70 % en poids (soit 0,706 mol/h de peroxyde d'hydrogène).

Le temps de passage dans ce réacteur est de 11 minutes ; le milieu étant maintenu à 50°C.

Le milieu réactionnel de ce réacteur est introduit en continu à l'aide d'une pompe dans un échangeur dans lequel il est chauffé à 80°C puis dans un réacteur tubulaire garni de mélangeurs Sulzer SMX de 57 mL de volume global (longueur = 200 mm; diamètre = 19 mm); ce réacteur étant calorifugé et maintenu en pression par un clapet taré à 10 bar en sortie. Le temps de passage dans l'échangeur est de l'ordre de 20 secondes.

Après une durée de stabilisation (environ ½ h), on dose les diphénols formés par chromatographie liquide haute performance et le peroxyde d'hydrogène par potentiométrie.

Les résultats obtenus à la sortie du réacteur tubulaire sont consignés dans le tableau (I).

Exemple 3 (comparatif)

Dans un réacteur de volume utile 30 mL muni d'une double enveloppe et équipé d'un système d'agitation type 4 pales inclinées, d'un réfrigérant ascendant, d'une arrivée d'azote et d'un dispositif de chauffage, on introduit à l'aide de pompes à 50°C en continu :

- 130 g/h de phénol contenant l'acide pyrophosphorique à raison de 400 ppm massique par rapport au phénol,
- l'acide perchlorique à raison de 400 ppm molaires par rapport au phénol
- 5,4 g/h de peroxyde d'hydrogène à 70 % en poids (soit 0,110 mol/h de peroxyde d'hydrogène).

Le temps de passage dans ce réacteur est de l'ordre de 14 minutes.

Le milieu réactionnel de ce réacteur est introduit en continu à l'aide d'une pompe dans un réacteur tubulaire garni de mélangeurs Sulzer SMX de 57 mL de volume global (longueur = 200 mm; diamètre = 19 mm), double-enveloppé et dont la température est fixée à 110°C. Cette température correspond à la moyenne des températures du réacteur adiabatique de l'exemple n°2.

Après une durée de stabilisation (environ 1 h ½ ), on dose les diphénols formés par chromatographie liquide haute performance et le peroxyde d'hydrogène par potentiométrie.

Les résultats obtenus à la sortie du réacteur tubulaire sont consignés dans le tableau (I).

Tableau (I)

| Réf. Exemple | 1 | 2 | 3 comparatif |
|---|---|---|---|
| Type de réacteur | 1 réacteur agité à 50°C + 1 réacteur piston adiabatique | 1 réacteur agité à 50°C + 1 réacteur piston adiabatique | 1 réacteur agité à 50°C + 1 réacteur piston isotherme 110°C |
| Catalyseur | $HClO_4$ | $HClO_4$ | $HClO_4$ |
| $HClO_4$ / PhOH (ppm mol) | 400 | 400 | 400 |
| Acide pyrophosphorique/ PhOH (ppm pds) | 400 | 400 | 400 |
| $H_2O_2$/PhOH (% mol) | 5,7 | 8,0 | 8,0 |
| Temps de passage (min) | 4 | 4 | 26 |
| Température d'entrée (°C) | 80 | 80 | 110 |

(suite)

| Réf. Exemple | 1 | 2 | 3 comparatif |
|---|---|---|---|
| Type de réacteur | 1 réacteur agité à 50°C + 1 réacteur piston adiabatique | 1 réacteur agité à 50°C + 1 réacteur piston adiabatique | 1 réacteur agité à 50°C + 1 réacteur piston isotherme 110°C |
| Température de sortie (°C) | 143 | 170 | 110 |
| TT PhOH | 5,1 % | 6,9 % | 7,0 % |
| RR HQ | 31 % | 27 % | 30 % |
| RR PC | 48 % | 45 % | 46 % |
| RR (diphénols) | 78 % | 72 % | 76 % |
| Production (diphénols) (g/h) | 46,1 | 56,1 | 9,2 |
| Ratio PC/HQ | 1,55 | 1,64 | 1,54 |
| TT $H_2O_2$ | 99 % | 99 % | 99 % |
| RT (diphénols) / $H_2O_2$ | 79 % | 72 % | 76 % |
| Charge thermique de refroidissement (kcal/h) | 0 | 0 | 2.7 |

Ce tableau montre que le fait de se placer en conditions adiabatiques plutôt qu'en isotherme permet de réduire de façon importante le temps de passage dans le réacteur.

La productivité est par conséquent fortement améliorée même si l'on observe une légère baisse de la sélectivité : pour un même réacteur, la réaction adiabatique permet de multiplier la production de diphénols par un facteur 6 par rapport au même essai en isotherme.

Enfin, la réaction adiabatique présente l'avantage de ne nécessiter aucun refroidissement contrairement au réacteur isotherme.

Exemple 4

Dans un réacteur de volume utile 50 mL muni d'une double enveloppe et équipé d'un système d'agitation type 4 pales inclinées, d'un réfrigérant ascendant, d'une arrivée d'azote et d'un système de régulation de la température, on introduit à l'aide de pompes à 50°C en continu :

- 182 g/h (1,94 mol/h) de phénol contenant l'acide pyrophosphorique à raison de 400 ppm massique par rapport au phénol,
- l'acide perchlorique à raison de 400 ppm molaires par rapport au phénol,
- 17,6 g/h de peroxyde d'hydrogène à 30 % en poids (soit 0,15 mol/h de peroxyde d'hydrogène).

Le temps de passage dans ce réacteur est de 15 minutes, le milieu est maintenu à 50°C.

Le milieu réactionnel de ce réacteur est introduit en continu à l'aide d'une pompe dans un échangeur dans lequel il est chauffé à 80°C puis dans un réacteur tubulaire garni de mélangeurs Sulzer SMX de 284 mL de volume global (longueur = 1000 mm; diamètre = 19 mm), ce réacteur étant calorifugé et maintenu en pression par un clapet taré à 10 bar en sortie. Le temps de passage dans l'échangeur est de l'ordre de 1 minute 30 secondes.

Après une durée de stabilisation (environ 3h), on dose les diphénols formés par chromatographie liquide haute performance et le peroxyde d'hydrogène par potentiométrie.

Les résultats obtenus à la sortie du réacteur tubulaire sont consignés dans le tableau (II).

Tableau (II)

| Référence exemple | 2 | 4 |
|---|---|---|
| Type de réacteur | 1 réacteur agité à 50°C + 1 réacteur piston adiabatique $H_2O_2$ 70% | 1 réacteur agité à 50°C + 1 réacteur piston adiabatique $H_2O_2$ 30% |
| Catalyseur | $HClO_4$ | $HClO_4$ |

(suite)

| Référence exemple | 2 | 4 |
|---|---|---|
| Type de réacteur | 1 réacteur agité à 50°C + 1 réacteur piston adiabatique $H_2O_2$ 70% | 1 réacteur agité à 50°C + 1 réacteur piston adiabatique $H_2O_2$ 30% |
| $HClO_4$/ PhOH (ppm mol) | 400 | 400 |
| Acide pyrophosphorique/ PhOH (ppm pds) | 400 | 400 |
| $H_2O_2$/PhOH (% mol) | 8,0 | 8,0 |
| Temps de passage (min) | 4 | 89 |
| Température d'entrée (°C) | 80 | 80 |
| Température de sortie (°C) | 170 | 170 |
| TT PhOH | 6,9 % | 6,9 % |
| RR HQ | 27 % | 28 % |
| RR PC | 45 % | 45 % |
| RR (diphénols) | 72 % | 73 % |
| Production (diphénols) (g/h) | 56,1 | 9,2 |
| Ratio PC/HQ | 1,64 | 1,61 |
| TT $H_2O_2$ | 99 % | 99 % |
| RT (diphénols) / $H_2O_2$ | 72 % | 73 % |
| Charge thermique de refroidissement (kcal/h) | 0 | 0 |

Ce tableau montre que l'utilisation d'une solution d'eau oxygénée à 30% poids augmente fortement le temps de réaction sans modification significative des sélectivités, par comparaison avec l'utilisation d'eau oxygénée à 70% poids.

## Revendications

1. Procédé d'hydroxylation d'un phénol ou d'un éther de phénol, par réaction dudit phénol ou éther de phénol, avec le peroxyde d'hydrogène, en présence d'un catalyseur acide fort homogène ayant un pKa dans l'eau inférieur à -0,1, **caractérisé par le fait qu'**il comprend les étapes suivantes mises en oeuvre successivement ou simultanément :

   - une première étape de mélange d'un phénol ou éther de phénol avec une solution de peroxyde d'hydrogène dans des conditions telles que la température soit suffisante pour que le phénol ou l'éther de phénol de départ reste liquide et que le taux de transformation du peroxyde d'hydrogène soit inférieur à 25 % en mol,
   - une deuxième étape consistant à effectuer la réaction d'hydroxylation du phénol ou de l'éther de phénol dans des conditions adiabatiques ; le catalyseur acide étant introduit à l'étape du mélange et/ou en début de réaction d'hydroxylation,
   - une troisième étape si nécessaire de récupération du produit hydroxylé.

2. Procédé selon la revendication 1, **caractérisé par le fait que** l'opération de mélange est conduite de telle sorte que le taux de transformation du peroxyde d'hydrogène soit compris entre 0,5 % et 25 % et plus préférentiellement entre 0,5 % et 15 % en mol.

3. Procédé selon l'une des revendications 1 et 2, **caractérisé par le fait que** la température de l'opération de mélange est choisie au plus égale à 85°C, de préférence comprise entre 45°C et 60°C.

**4.** Procédé selon l'une des revendications 1 à 3, **caractérisé par le fait que** le mélange est effectué sous pression atmosphérique ou à des pressions supérieures, de préférence entre 1 et 200 bar absolus.

**5.** Procédé selon l'une des revendications 1 à 4, **caractérisé par le fait que** l'on fait le mélange en introduisant par voie séparée : le composé phénolique éventuellement additionné d'un agent complexant ; éventuellement tout ou partie du catalyseur acide ; la solution de peroxyde d'hydrogène ; et éventuellement un co-catalyseur.

**6.** Procédé selon l'une des revendications 1 à 5, **caractérisé par le fait que** la réaction d'hydroxylation est conduite dans un réacteur présentant la caractéristique d'être isolé thermiquement de l'extérieur et qui est un réacteur en matériau isolant et/ou calorifugé.

**7.** Procédé selon la revendication 6, **caractérisé par le fait que** le réacteur est :

- un réacteur en polymère PVDF (polyfluorure de vinylidène), PVC (polychlorure de vinyle, PTFE (polytétrafluoroéthylène) chargé ou non de préférence par du carbone ou des fibres de verre ; en verre ou en acier vitrifié,
- et/ou un réacteur calorifugé par de la laine de verre, laine de roche, mousse synthétique isolante, de préférence mousse de polyuréthane,
- le calorifuge étant éventuellement recouvert par un revêtement métallique.

**8.** Procédé selon la revendication 1, **caractérisé par le fait que**, lorsque l'on conduit le mélange des réactifs et la réaction d'hydroxylation simultanément, on préchauffe au moins le composé phénolique à une température supérieure à 70°C, de préférence comprise entre 70°C et 85°C.

**9.** Procédé selon l'une des revendications 1 à 7, **caractérisé par le fait que** l'on effectue le mélange des réactifs dans un dispositif de mélange pourvu d'une agitation et de moyens de chauffage et l'on réalise l'hydroxylation dans des conditions adiabatiques dans un réacteur à écoulement piston.

**10.** Procédé selon la revendication 8, **caractérisé par le fait que** l'on conduit l'étape de mélange des réactifs et l'étape d'hydroxylation dans des conditions adiabatiques dans un réacteur à écoulement piston.

**11.** Procédé selon l'une des revendications 1 à 7, **caractérisé par le fait que** l'on effectue le mélange des réactifs dans un dispositif de mélange pourvu d'une agitation et de moyens de chauffage et l'on réalise la deuxième étape d'hydroxylation dans une succession d'au moins deux réacteurs à écoulement piston maintenus dans des conditions adiabatiques et séparés par un échangeur thermique.

**12.** Procédé selon l'une des revendications 1 à 7 **caractérisé par le fait que** l'on effectue le mélange des réactifs dans un dispositif de mélange pourvu d'une agitation et de moyens de chauffage et l'on réalise la deuxième étape d'hydroxylation dans un faisceau de réacteurs à écoulement piston montés en parallèle.

**13.** Procédé selon l'une des revendications 9, 11 et 12, **caractérisé par le fait que** le dispositif de mélange est un réacteur agité mécaniquement, de préférence un réacteur à turbine à pales droites ou inclinées ou à l'hélice marine ou à mobile "hydrofoil" ; un réacteur agité mécaniquement ou non avec une boucle de circulation d'une fraction ou totalité du contenu dudit réacteur, à l'aide d'une pompe sur boucle externe ; un mélangeur dynamique de préférence un mélangeur à jets tangentiels, là jets d'impact ou des éjecteurs ; un mélangeur statique, de préférence un mélangeur statique (Sulzer SMX, Kenics, etc...), un lit de billes ou particules en vrac, des mousses métalliques ou céramiques.

**14.** Procédé selon la revendication 13, **caractérisé par le fait que** la surface d'échange pour assurer le transfert thermique peut être augmentée par des serpentins ou des plaques présentes à l'intérieur du réacteur ou par l'intermédiaire d'un fluide caloporteur circulant dans une double enveloppe.

**15.** Procédé selon l'une des revendications 9 à 12, **caractérisé par le fait que** le réacteur à écoulement piston est un réacteur tubulaire qui présente un rapport longueur / diamètre supérieur à 3, de préférence compris entre 4 et 30 et encore plus préférentiellement compris entre 5 et 10.

**16.** Procédé selon l'une des revendications 9 à 12 et 15, **caractérisé par le fait que** le réacteur à écoulement piston est un réacteur tubulaire disposé à l'horizontale, à la verticale ou encore incliné.

**17.** Procédé selon l'une des revendications 9 à 12, 15 et 16, **caractérisé par le fait que** le réacteur comporte des internes lorsque l'écoulement dans le réacteur présente un nombre de Reynolds inférieur à 5000.

**18.** Procédé selon l'une des revendications 9 à 12, 15 et 16, **caractérisé par le fait que** le réacteur ne comporte pas d'internes lorsque l'écoulement dans le réacteur présente un nombre de Reynolds supérieur ou égal à 5000.

**19.** Procédé selon l'une des revendications 9 à 12, 15 et 16, **caractérisé par le fait que** le réacteur sans internes est structuré lorsque le nombre de Reynolds est inférieur à 5000 ; ledit réacteur structuré pouvant être équipé partiellement d'internes.

**20.** Procédé selon la revendication 17, **caractérisé par le fait que** le réacteur est rempli tout ou partie par des internes qui peuvent être des internes en vrac, consistant en de petits objets par exemple sous forme d'anneaux, de selles, de boules ou cylindres creux ou le réacteur comprend des internes structurés : clavettes, mélangeurs statiques, chicanes.

**21.** Procédé selon l'une des revendications 9 à 12, 15 à 20, **caractérisé par le fait que** le réacteur à écoulement piston est un réacteur tubulaire formé de tubes concentriques ou un réacteur en forme de colonne.

**22.** Procédé selon la revendication 1, **caractérisé par le fait que** le substrat phénolique répond à la formule générale (I) :

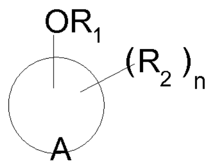

(I)

dans laquelle :

- A symbolise un cycle benzénique ou naphtalénique,
- $R_1$ représente un atome d'hydrogène, un groupe alkyle, cycloalkyle, aryle, aralkyle,
- $R_2$ représente un atome d'hydrogène ou un ou plusieurs substituants, identiques ou différents,
- n, nombre de substituant par cycle aromatique, est un nombre inférieur ou égal à 4.

**23.** Procédé selon la revendication 22, **caractérisé par le fait que** le substrat phénolique répond à la formule générale (Ia) :

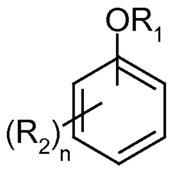

(Ia)

dans ladite formule :

- n est un nombre de 0 à 4, de préférence égal à 0, 1 ou 2,
- $R_1$ représente un atome d'hydrogène, un groupe alkyle, cycloalkyle, aryle, aralkyle,
- $R_2$, identiques ou différents, représentent un groupe alkyle, un groupe alkoxy, un groupe hydroxyle, un atome d'halogène, un groupe halogéno- ou perhalogénoalkyle.

**24.** Procédé selon la revendication 23, **caractérisé par le fait que** le substrat phénolique est le phénol, l'o-crésol, le m-crésol, le p-crésol, l'anisole, le phénétole, le 2-méthoxyphénol (le gaïacol), le 2-éthoxyphénol (le guétol).

**25.** Procédé selon la revendication 1, **caractérisé par le fait que** le catalyseur acide est un acide protonique fort présentant un pKa dans l'eau inférieur à - 1,0 ou un mélange d'acides protoniques.

**26.** Procédé selon la revendication 25, **caractérisé par le fait que** le catalyseur acide est l'acide sulfurique, l'acide perchlorique, l'acide méthanesulfonique, trifluorométhanesulfonique, toluènesulfonique, phénolsulfonique, bis-trifluorométhanesulfonimide, ou un mélange d'acide perchlorique et d'acide sulfurique ; d'acide perchlorique et d'acide 4-hydroxybenzènesulfonique ; d'acide trifluorométhanesulfonique et d'acide 4-hydroxybenzènesulfonique ; d'acide bis-trifluorométhanesulfonimide et d'acide 4-hydroxybenzènesulfonique.

**27.** Procédé selon la revendication 1, **caractérisé par le fait que** l'on ajoute en outre un co-catalyseur qui est un composé cétonique qui répond à la formule (II) :

$$R_a\text{-CO-X-}R_b \qquad \text{(II)}$$

dans ladite formule (II) :

- $R_a$ et $R_b$, identiques ou différents, représentent des groupes hydrocarbonés ayant de 1 à 30 atomes de carbone ou forment ensemble un groupe divalent, éventuellement substitués par un ou plusieurs atomes d'halogène ou groupes fonctionnels stables dans les conditions de la réaction,
- X représente un lien valentiel, un groupe -CO-, un groupe -CHOH ou un groupe $-(R)_n-$ : R représentant un groupe alkylène ayant de préférence de 1 à 4 atomes de carbone et n est un nombre entier choisi entre 1 et 16.

**28.** Procédé selon la revendication 27, **caractérisé par le fait que** le composé cétonique répond à la formule (IIa) suivante :

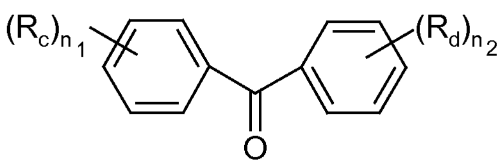

(IIa)

dans ladite formule (IIa) :

- $R_c$ et $R_d$, identiques ou différents représentent un atome d'hydrogène ou un substituant, de préférence un groupe électro-donneur,
- $n_1$, $n_2$, identiques ou différents est un nombre égal à 0, 1, 2 ou 3,
- éventuellement les deux atomes de carbone situés en position $\alpha$ par rapport aux deux atomes de carbone portant le groupe -CO peuvent être liés ensemble par un lien valentiel ou par un groupe $-CH_2-$ formant ainsi un cycle cétonique qui peut être saturé mais aussi insaturé.

**29.** Procédé selon la revendication 1, **caractérisé par le fait que** l'on met en oeuvre une solution aqueuse de peroxyde d'hydrogène ayant une concentration en $H_2O_2$ d'au moins 20 % en poids, de préférence, comprise entre 30 % et 90 % en poids et encore plus préférentiellement comprise entre 30 % et 70 % en poids.

**30.** Procédé selon la revendication 1, **caractérisé par le fait que** l'on opère en présence d'un agent complexant des ions de métaux de transition, stables dans les conditions de réaction tels que les acides phosphoriques, les acides pyrophosphoriques, les acides phosphoniques et leurs esters-acides.

**Patentansprüche**

**1.** Verfahren zur Hydroxylierung eines Phenols oder eines Phenolethers, durch Umsetzen des Phenols oder Phenolethers mit Wasserstoffperoxid in Anwesenheit eines homogenen starken Säurekatalysators mit einem pKa in Wasser von weniger als -0,1,
**dadurch gekennzeichnet, dass** dieses die folgenden Schritte umfasst, die aufeinanderfolgend oder gleichzeitig durchgeführt werden:

- einen ersten Schritt des Mischens eines Phenols oder Phenolethers mit einer Wasserstoffperoxid-Lösung unter derartigen Bedingungen, dass die Temperatur ausreicht, damit das Ausgangs-Phenol oder der Ausgangs-Phenolether flüssig bleibt und dass die Transformationsrate von Wasserstoffperoxid unter 25 Mol-% ist,

- einen zweiten Schritt, der aus dem Bewirken der Hydroxylierungsreaktion des Phenols oder des Phenolethers unter adiabatischen Bedingungen besteht; wobei der Säurekatalysator in dem Schritt des Mischens und/oder am Beginn der Hydroxylierungsreaktion eingebracht wird,
- erforderlichenfalls einen dritten Schritt der Gewinnung des hydroxylierten Produkts.

**2.** Verfahren nach Anspruch 1, **dadurch gekennzeichnet, dass** der Mischvorgang derart durchgeführt wird, dass die Transformationsrate von Wasserstoffperoxid zwischen 0,5 Mol-% und 25 Mol-% und bevorzugter zwischen 0,5 Mol-% und 15 Mol-% liegt.

**3.** Verfahren nach einem der Ansprüche 1 und 2, **dadurch gekennzeichnet, dass** die Temperatur des Mischvorgangs höchstens gleich 85°C, vorzugsweise zwischen 45°C und 60°C gewählt wird.

**4.** Verfahren nach einem der Ansprüche 1 bis 3, **dadurch gekennzeichnet, dass** das Mischen unter Atmosphärendruck oder bei höheren Drücken, vorzugsweise zwischen 1 und 200 bar absolut bewirkt wird.

**5.** Verfahren nach einem der Ansprüche 1 bis 4, **dadurch gekennzeichnet, dass** die Mischung hergestellt wird, indem auf getrenntem Weg eingebracht werden: die Phenol-Verbindung, der gegebenenfalls ein Komplexbildner zugesetzt wird; gegebenenfalls der gesamte oder ein Teil des Säurekatalysators; die Wasserstoffperoxid-Lösung; und gegebenenfalls ein Co-Katalysator.

**6.** Verfahren nach einem der Ansprüche 1 bis 5, **dadurch gekennzeichnet, dass** die Hydroxylierungsreaktion in einem Reaktor durchgeführt wird, der das Merkmal aufweist, thermisch gegen die Außenseite isoliert zu sein, und der ein Reaktor aus Isolier- und/oder wärmegedämmtem Material ist.

**7.** Verfahren nach Anspruch 6, **dadurch gekennzeichnet, dass** der Reaktor ist:

- ein Reaktor aus Polymer PVDF (Polyvinylidenfluorid), PVC (Polyvinylchlorid), PTFE (Polytetrafluorethylen), vorzugsweise gegebenenfalls mit Kohlenstoff oder Glasfasern beladen; aus Glas oder glasiertem Stahl,
- und/oder ein Reaktor, der mit Glaswolle, Steinwolle, einem synthetischen Isolierschaum, vorzugsweise Polyurethanschaum, wärmegedämmt ist,
- wobei die Wärmedämmung gegebenenfalls durch eine metallische Verkleidung abgedeckt wird.

**8.** Verfahren nach Anspruch 1, **dadurch gekennzeichnet, dass**, wenn das Mischen der Reagenzien und die Hydroxylierungsreaktion gleichzeitig durchgeführt werden, mindestens die Phenol-Verbindung auf eine Temperatur von mehr als 70°C, vorzugsweise zwischen 70°C und 85°C, erhitzt wird.

**9.** Verfahren nach einem der Ansprüche 1 bis 7, **dadurch gekennzeichnet, dass** das Mischen der Reagenzien in einer Mischvorrichtung bewirkt wird, die mit einem Rührwerk und Heizmitteln versehen ist, und die Hydroxylierung unter adiabatischen Bedingungen in einem Pfropfenströmungsreaktor durchgeführt wird.

**10.** Verfahren nach Anspruch 8, **dadurch gekennzeichnet, dass** der Schritt des Mischens der Reagenzien und der Schritt der Hydroxylierung unter adiabatischen Bedingungen in einem Pfropfenströmungsreaktor durchgeführt werden.

**11.** Verfahren nach einem der Ansprüche 1 bis 7, **dadurch gekennzeichnet, dass** das Mischen der Reagenzien in einer Mischvorrichtung bewirkt wird, die mit einem Rührwerk und Heizmitteln versehen ist, und dass der zweite Schritt der Hydroxylierung in einer Folge von mindestens zwei Pfropfenströmungsreaktoren durchgeführt wird, die unter adiabatischen Bedingungen gehalten werden und durch einen Wärmetauscher getrennt sind.

**12.** Verfahren nach einem der Ansprüche 1 bis 7, **dadurch gekennzeichnet, dass** das Mischen der Reagenzien in einer Mischvorrichtung bewirkt wird, die mit einem Rührwerk und Heizmitteln versehen ist, und dass der zweite Schritt der Hydroxylierung in einem Bündel von Pfropfenströmungsreaktoren durchgeführt wird, die parallel montiert sind.

**13.** Verfahren nach einem der Ansprüche 9, 11 und 12, **dadurch gekennzeichnet, dass** die Mischvorrichtung ein mechanisch bewegter Reaktor ist, vorzugsweise ein Reaktor mit einer Turbine mit geraden oder geneigten Schaufeln oder mit einer Schiffsschraube oder mit einer mobilen "Tragfläche"; gegebenenfalls ein mechanisch bewegter Reaktor mit einem Zirkulationskreislauf einer Fraktion oder der Gesamtheit des Inhalts des Reaktors, mit Hilfe einer

Pumpe mit externem Kreislauf; ein dynamischer Mischer, vorzugsweise ein Mischer mit tangentialen Strahlen, hier Aufprallstrahlen oder Ejektoren; ein statischer Mischer, vorzugsweise ein statischer Mischer (Sulzer SMX, Kenics usw.), ein Kugelbett oder Schüttgutteilchen, metallische oder keramische Schäume.

**14.** Verfahren nach Anspruch 13, **dadurch gekennzeichnet, dass** die Austauschfläche, um den Wärmetransfer zu gewährleisten, durch Serpentinen oder Platten, die im Inneren des Reaktors angeordnet sind, oder mit Hilfe eines Wärmeübertragungsfluids, das in einem Doppelmantel zirkuliert, erhöht werden kann.

**15.** Verfahren nach einem der Ansprüche 9 bis 12, **dadurch gekennzeichnet, dass** der Pfropfenströmungsreaktor ein rohrförmiger Reaktor ist, der ein Verhältnis Länge/Durchmesser von mehr als 3, vorzugsweise zwischen 4 und 30 und noch bevorzugter zwischen 5 und 10 aufweist.

**16.** Verfahren nach einem der Ansprüche 9 bis 12 und 15, **dadurch gekennzeichnet, dass** der Pfropfenströmungsreaktor ein rohrförmiger Reaktor ist, der horizontal, vertikal oder auch geneigt angeordnet ist.

**17.** Verfahren nach einem der Ansprüche 9 bis 12, 15 und 16, **dadurch gekennzeichnet, dass** der Reaktor Inneneinrichtungen umfasst, wenn die Strömung in dem Reaktor eine Reynolds-Zahl von weniger als 5000 aufweist.

**18.** Verfahren nach einem der Ansprüche 9 bis 12, 15 und 16, **dadurch gekennzeichnet, dass** der Reaktor keine Inneneinrichtungen umfasst, wenn die Strömung in dem Reaktor eine Reynolds-Zahl von größer oder gleich 5000 aufweist.

**19.** Verfahren nach einem der Ansprüche 9 bis 12, 15 und 16, **dadurch gekennzeichnet, dass** der Reaktor ohne Inneneinrichtungen strukturiert wird, wenn die Reynolds-Zahl kleiner als 5000 ist; wobei der strukturierte Reaktor teilweise mit Inneneinrichtungen ausgestattet werden kann.

**20.** Verfahren nach Anspruch 17, **dadurch gekennzeichnet, dass** der Reaktor vollständig oder teilweise mit Inneneinrichtungen gefüllt wird, die Inneneinrichtungen aus Schüttgut, das aus kleinen Objekten beispielsweise in Form von Ringen, Satteln, Kugeln oder Hohlzylindern besteht, sein können, oder dass der Reaktor innere Strukturen umfasst: Keile, statische Mischer, Ablenkbleche.

**21.** Verfahren nach einem der Ansprüche 9 bis 12, 15 bis 20, **dadurch gekennzeichnet, dass** der Pfropfenströmungsreaktor ein rohrförmiger Reaktor, der aus konzentrischen Rohren gebildet ist, oder ein Reaktor in Form einer Säule ist.

**22.** Verfahren nach Anspruch 1, **dadurch gekennzeichnet, dass** das Phenol-Substrat die allgemeine Formel (I) aufweist:

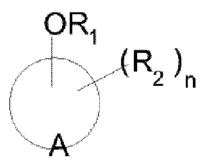

(I)

wobei:

- A einen Benzol- oder Naphtalinring bedeutet,
- $R_1$ ein Wasserstoffatom, eine Alkyl-, Cycloalkyl-, Aryl-, Aralkyl-Gruppe darstellt,
- $R_2$ ein Wasserstoffatom oder einen oder mehrere gleiche oder verschiedene Substituenten darstellt,
- n, die Anzahl der Substituenten pro aromatischen Ring, eine Zahl kleiner oder gleich 4 ist.

**23.** Verfahren nach Anspruch 22, **dadurch gekennzeichnet, dass** das Phenol-Substrat die allgemeine Formel (Ia) aufweist:

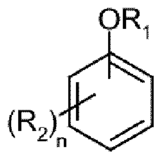

(Ia)

wobei in der Formel:

- n eine Zahl von 0 bis 4, vorzugsweise gleich 0, 1 oder 2 ist,
- $R_1$ ein Wasserstoffatom, eine Alkyl-, Cycloalkyl-, Aryl-, Aralkyl-Gruppe darstellt,
- $R_2$, die gleich oder verschieden sein können, eine Alkyl-Gruppe, eine Alkoxy-Gruppe, eine Hydroxyl-Gruppe, ein Wasserstoffatom, eine Halogen- oder Perhalogenalkyl-Gruppe darstellen.

**24.** Verfahren nach Anspruch 23, **dadurch gekennzeichnet, dass** das Phenol-Substrat Phenol, o-Kresol, m-Kresol, p-Kresol, Anisol, Phenetol, 2-Methoxyphenol (Guajacol), 2-Ethoxyphenol (Guaethol) ist.

**25.** Verfahren nach Anspruch 1, **dadurch gekennzeichnet, dass** der Säurekatalysator eine starke Protonensäure mit einem pKa in Wasser von weniger als -1,0 oder eine Mischung von Protonensäuren ist.

**26.** Verfahren nach Anspruch 25, **dadurch gekennzeichnet, dass** der Säurekatalysator Schwefelsäure, Perchlorsäure, Methansulfonsäure, Trifluormethansulfonsäure, Toluolsulfonsäure, Phenolsulfonsäure, Bis-Trifluormethansulfonimid oder eine Mischung von Perchlorsäure und Schwefelsäure; von Perchlorsäure und 4-Hydroxybenzolsulfonsäure; von Trifluormethansulfonsäure und 4-Hydroxybenzolsulfonsäure; von Bis-Trifluormethansulfonimid und 4-Hydroxybenzolsulfonsäure ist.

**27.** Verfahren nach Anspruch 1, **dadurch gekennzeichnet, dass** außerdem ein Co-Katalysator zugesetzt wird, der eine ketonische Verbindung ist, welche die allgemeine Formel (II) aufweist:

$$R_a\text{-CO-X-}R_b \qquad \text{(II)}$$

wobei in der Formel (II):

- $R_a$ und $R_b$, die gleich oder verschieden sind, Kohlenwasserstoffgruppen mit 1 bis 30 Kohlenstoffatomen darstellen oder miteinander eine divalente Gruppe bilden, gegebenenfalls substituiert durch ein oder mehrere Halogenatome oder unter den Reaktionsbedingungen stabile funktionelle Gruppen,
- X eine Valenzverbindung, eine Gruppe -CO-, eine Gruppe -CHOH oder eine Gruppe $-(R)_n$-darstellt, wobei R eine Alkylen-Gruppe mit vorzugsweise 1 bis 4 Kohlenstoffatomen darstellt, und n eine ganze Zahl ist, die zwischen 1 und 16 ausgewählt wird.

**28.** Verfahren nach Anspruch 27, **dadurch gekennzeichnet, dass** die ketonische Verbindung die folgende allgemeine Formel (IIa) aufweist:

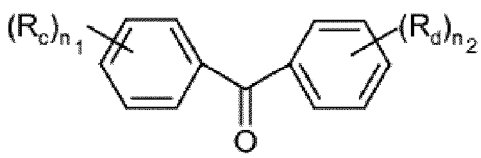

(IIa)

wobei in der Formel (IIa):

- $R_c$ und $R_d$, die gleich oder verschieden sind, ein Wasserstoffatom oder einen Substituenten, vorzugsweise eine Elektronendonator-Gruppe, darstellen,
- $n_1$, $n_2$, die gleich oder verschieden sind, eine Zahl gleich 0, 1, 2 oder 3 sind,
- gegebenenfalls die beiden Kohlenstoffatome, die in Stellung $\alpha$ in Bezug auf zwei Kohlenstoffatome angeordnet sind, welche die Gruppe -CO tragen, miteinander durch eine Valenzverbindung oder durch eine Gruppe $-CH_2$- verbunden sein können, die so einen Ketonring bilden, der gesättigt, jedoch auch ungesättigt sein kann.

**29.** Verfahren nach Anspruch 1, **dadurch gekennzeichnet, dass** eine wässerige Lösung von Wasserstoffperoxid mit einer Konzentration von $H_2O_2$ von mindestens 20 Gew.-%, vorzugsweise zwischen 30 Gew.-% und 90 Gew.-% oder noch bevorzugter zwischen 30 Gew.-% und 70 Gew.-% hergestellt wird.

**30.** Verfahren nach Anspruch 1, **dadurch gekennzeichnet, dass** in Anwesenheit eines Komplexbildners von Übergangsmetallionen gearbeitet wird, die unter den Reaktionsbedingungen stabil sind, wie Phosphorsäuren, Pyrophosphorsäuren, Phosphonsäuren und deren Säureester.

## Claims

**1.** Process for hydroxylating a phenol or a phenol ether, by reacting said phenol or phenol ether with hydrogen peroxide, in the presence of a homogeneous strong acid catalyst having a pKa in water of less than -0.1, **characterized in that** it comprises the following steps performed successively or simultaneously:

- a first step of mixing a phenol or a phenol ether with a hydrogen peroxide solution under conditions such that the temperature is sufficient for the starting phenol or phenol ether to remain liquid and for the degree of conversion of the hydrogen peroxide to be less than 25 mol%,
- a second step consisting in performing the hydroxylation reaction of the phenol or phenol ether under adiabatic conditions; the acid catalyst being introduced into the mixing step and/or at the start of the hydroxylation reaction,
- a third step, if necessary, of recovery of the hydroxylated product.

**2.** Process according to Claim 1, **characterized in that** the mixing operation is performed such that the degree of conversion of the hydrogen peroxide is between 0.5 mol% and 25 mol% and more preferentially between 0.5 mol% and 15 mol%.

**3.** Process according to either of Claims 1 and 2, **characterized in that** the temperature of the mixing operation is chosen to be not more than 85°C and preferably between 45°C and 60°C.

**4.** Process according to one of Claims 1 to 3, **characterized in that** the mixing is performed at atmospheric pressure or at higher pressures, preferably between 1 and 200 bar absolute.

**5.** Process according to one of Claims 1 to 4, **characterized in that** the mixing is performed by separately introducing: the phenolic compound optionally supplemented with a complexing agent; optionally all or part of the acid catalyst; the hydrogen peroxide solution; and optionally a cocatalyst.

**6.** Process according to one of Claims 1 to 5, **characterized in that** the hydroxylation reaction is performed in a reactor which has the characteristic of being thermally insulated from the exterior and which is a reactor made of an insulating material and/or lagged.

**7.** Process according to Claim 6, **characterized in that** the reactor is:

- a reactor made of PVDF (polyvinylidene fluoride), PVC (polyvinyl chloride) or PTFE (polytetrafluoroethylene) polymer, optionally filled, preferably with carbon or glass fiber; made of vitrified steel or glass,
- and/or a reactor lagged with glass wool, rockwool or insulating synthetic foam, preferably polyurethane foam,
- the lagging optionally being covered with a metallic coating.

**8.** Process according to Claim 1, **characterized in that**, when the mixing of the reagents and the hydroxylation reaction are performed simultaneously, at least the phenolic compound is preheated to a temperature above 70°C, preferably between 70°C and 85°C.

**9.** Process according to one of Claims 1 to 7, **characterized in that** the reagents are mixed together in a mixing device equipped with a stirrer and heating means, and the hydroxylation is performed under adiabatic conditions in a plug-flow reactor.

**10.** Process according to Claim 8, **characterized in that** the step of mixing of the reagents and the hydroxylation step are performed under adiabatic conditions in a plug-flow reactor.

**11.** Process according to one of Claims 1 to 7, **characterized in that** the reagents are mixed together in a mixing device equipped with a stirrer and heating means, and the hydroxylation second step is performed in a succession of at least two plug-flow reactors maintained under adiabatic conditions and separated by a heat exchanger.

**12.** Process according to one of Claims 1 to 7, **characterized in that** the reagents are mixed together in a mixing device equipped with a stirrer and heating means, and the hydroxylation second step is performed in an array of plug-flow reactors mounted in parallel.

**13.** Process according to either of Claims 9, 11 and 12, **characterized in that** the mixing device is a mechanically stirred reactor, preferably a turbine reactor with straight or inclined paddles or with a marine impeller or a mobile "hydrofoil"; a reactor, mechanically stirred or not, with a loop for circulation of a fraction or all of the contents of said reactor, with the aid of a pump on an external loop; a dynamic mixer, preferably a mixer with tangential jets, with impact jets or ejectors; a static mixer, preferably a static mixer (Sulzer SMX, Kenics, etc.), a bulk bed of beads or particles, metallic or ceramic foams.

**14.** Process according to Claim 13, **characterized in that** the exchange surface for the heat transfer may be increased by means of coils or plates present inside the reactor or via a heat-exchange fluid circulating in a jacket.

**15.** Process according to one of Claims 9 to 12, **characterized in that** the plug-flow reactor is a tubular reactor which has a length/diameter ratio of greater than 3, preferably between 4 and 30 and even more preferentially between 5 and 10.

**16.** Process according to one of Claims 9 to 12 and 15, **characterized in that** the plug-flow reactor is a tubular reactor arranged horizontally, vertically or inclined.

**17.** Process according to one of Claims 9 to 12, 15 and 16, **characterized in that** the reactor comprises baffles when the flow in the reactor has a Reynolds number of less than 5000.

**18.** Process according to one of Claims 9 to 12, 15 and 16, **characterized in that** the reactor does not comprise baffles when the flow in the reactor has a Reynolds number of greater than or equal to 5000.

**19.** Process according to one of Claims 9 to 12, 15 and 16, **characterized in that** the baffle-free reactor is structured when the Reynolds number is less than 5000; said structured reactor possibly being partially equipped with baffles.

**20.** Process according to Claim 17, **characterized in that** the reactor is totally or partially filled with baffles, which may be bulk baffles, consisting of small objects, for example in the form of rings, stools, balls or cylinders which are hollow, or the reactor comprises structured baffles: pins, static mixers, chicanes.

**21.** Process according to one of Claims 9 to 12 and 15 to 20, **characterized in that** the plug-flow reactor is a tubular reactor formed from concentric tubes or a reactor in the form of a column.

**22.** Process according to Claim 1, **characterized in that** the phenolic substrate corresponds to the general formula (I):

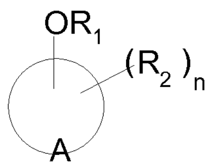

(I)

in which:

- A symbolizes a benzene or naphthalene ring,
- $R_1$ represents a hydrogen atom or an alkyl, cycloalkyl, aryl or aralkyl group,
- $R_2$ represents a hydrogen atom or one or more identical or different substituents,
- n, number of substituents per aromatic ring, is a number less than or equal to 4.

**23.** Process according to Claim 22, **characterized in that** the phenolic substrate corresponds to the general formula (Ia) :

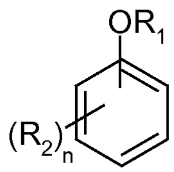

(Ia)

in said formula:

- n is a number from 0 to 4 and preferably equal to 0, 1, or 2,
- $R_1$ represents a hydrogen atom or an alkyl, cycloalkyl, aryl or aralkyl group,
- $R_2$, which may be identical or different, represent an alkyl group, an alkoxy group, a hydroxyl group, a halogen atom or a haloalkyl or perhaloalkyl group.

**24.** Process according to Claim 23, **characterized in that** the phenolic substrate is phenol, o-cresol, m-cresol, p-cresol, anisole, phenetole, 2-methoxyphenol (guaiacol) or 2-ethoxyphenol (guetol).

**25.** Process according to Claim 1, **characterized in that** the acid catalyst is a strong protic acid with a pKa in water of less than -1.0, or a mixture of protic acids.

**26.** Process according to Claim 25, **characterized in that** the acid catalyst is sulfuric acid, perchloric acid, methanesulfonic acid, trifluoromethanesulfonic acid, toluenesulfonic acid, phenolsulfonic acid, bis-trifluoromethanesulfonimide or a mixture of perchloric acid and sulfuric acid; of perchloric acid and 4-hydroxybenzenesulfonic acid; of trifluoromethanesulfonic acid and 4-hydroxybenzenesulfonic acid; of bis-trifluoromethanesulfonimide and 4-hydroxybenzenesulfonic acid.

**27.** Process according to Claim 1, **characterized in that** a cocatalyst is also added, which is a ketone compound corresponding to formula (II):

$$R_a - CO - X - R_b \qquad (II)$$

in said formula (II):

- $R_a$ and $R_b$, which may be identical or different, represent hydrocarbon-based groups containing from 1 to 30 carbon atoms or together form a divalent group, optionally substituted with one or more halogen atoms or functional groups that are stable under the reaction conditions,
- X represents a valency bond, a -CO- group, a - CHOH group or a group $-(R)_n-$: R representing an alkylene group preferably containing from 1 to 4 carbon atoms and n is an integer chosen between 1 and 16.

**28.** Process according to Claim 27, **characterized in that** the ketone compound corresponds to formula (IIa) below:

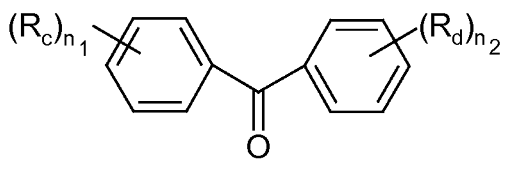

(IIa)

in said formula (IIa):

- $R_c$ and $R_d$, which may be identical or different, represent a hydrogen atom or a substituent, preferably an electron-donating group,
- $n_1$ and $n_2$, which may be identical or different, represent a number equal to 0, 1, 2 or 3,
- optionally, the two carbon atoms located $\alpha$ to the two carbon atoms bearing the -CO group may be linked together via a valency bond or via a-$CH_2$- group, thus forming a ketone ring, which may be saturated, but also unsaturated.

**29.** Process according to Claim 1, **characterized in that** an aqueous hydrogen peroxide solution having an $H_2O_2$ concentration of at least 20% by weight, preferably between 30% and 90% by weight and even more preferentially between 30% and 70% by weight is used.

**30.** Process according to Claim 1, **characterized in that** it is performed in the presence of an agent for complexing transition metal ions, which is stable under the reaction conditions, such as phosphoric acids, pyrophosphoric acids, phosphonic acids and acid esters thereof.

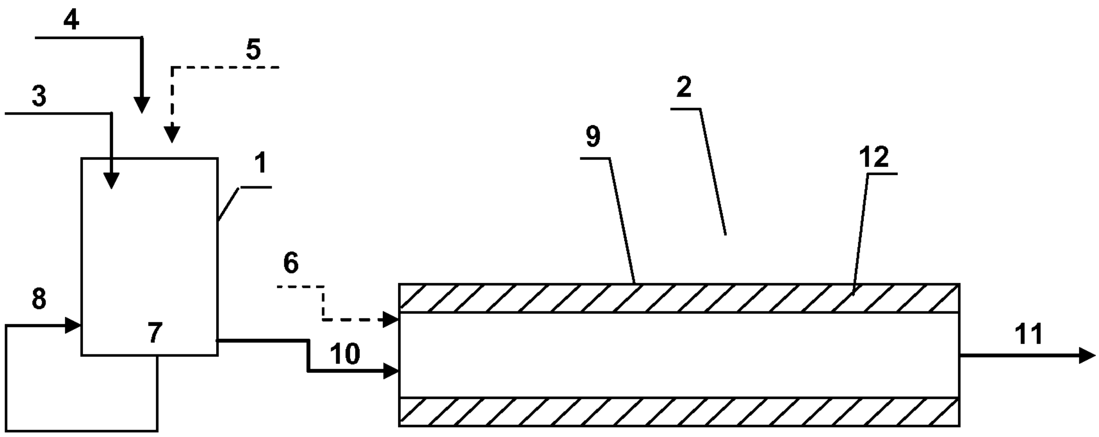


Figure 1

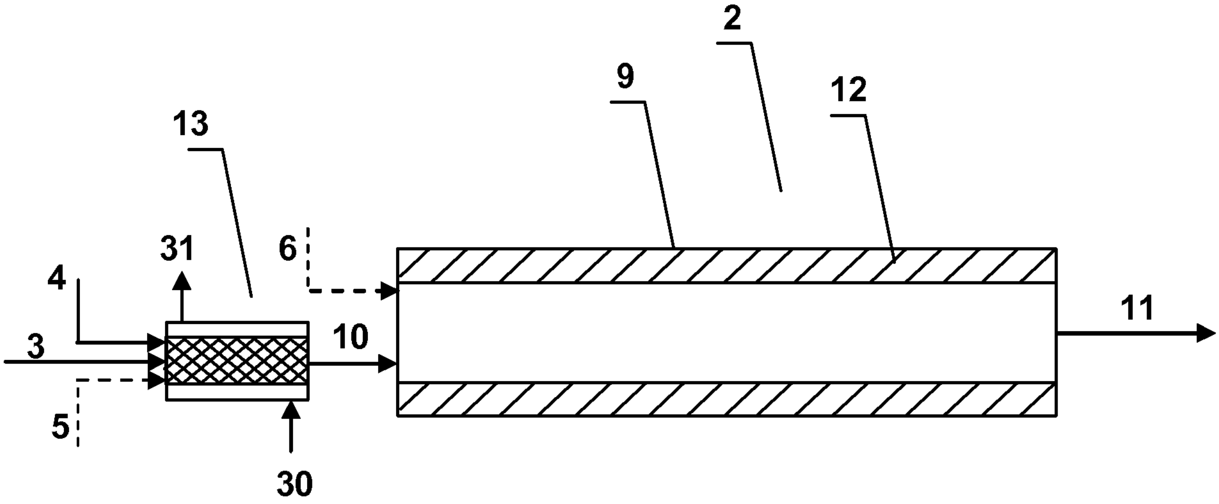


Figure 2

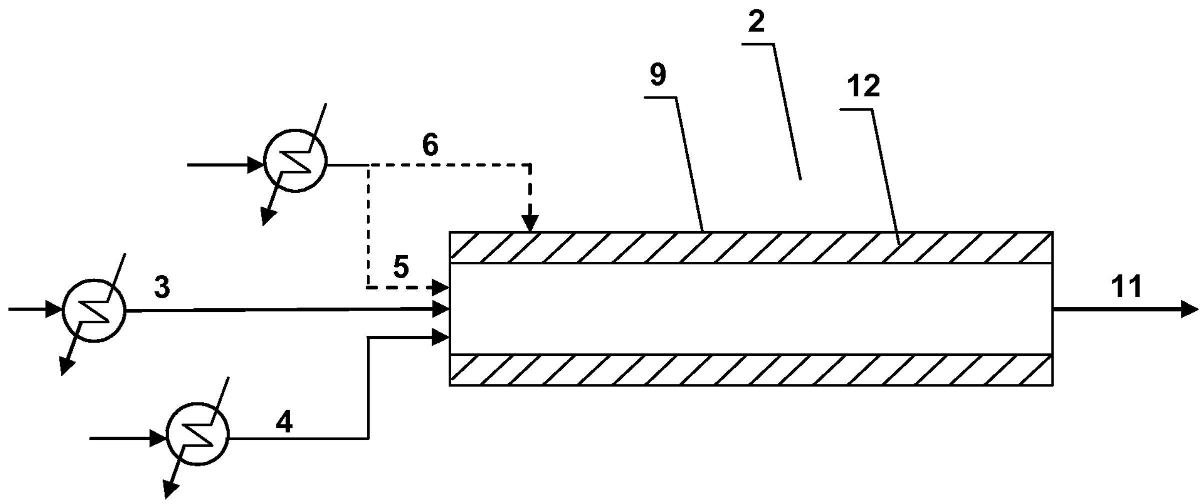


Figure 3

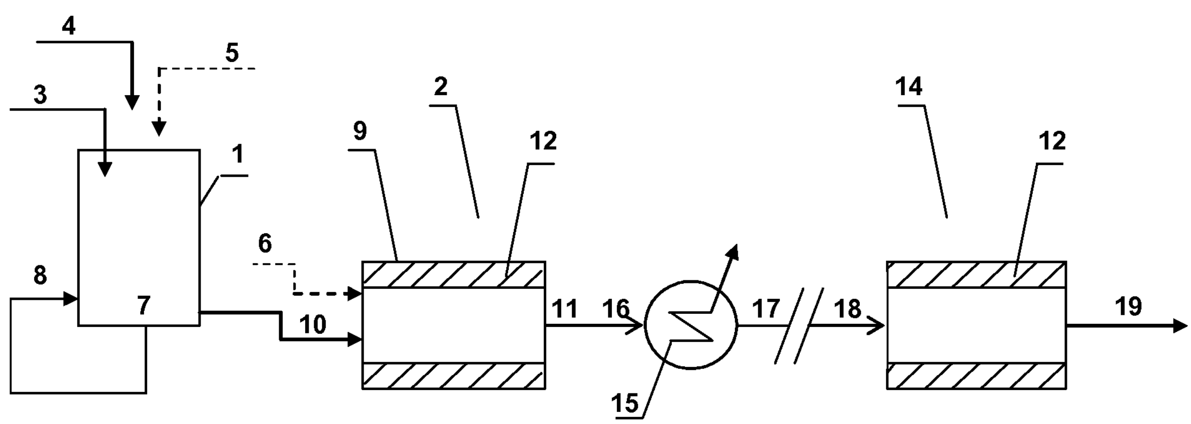


**Figure 4**

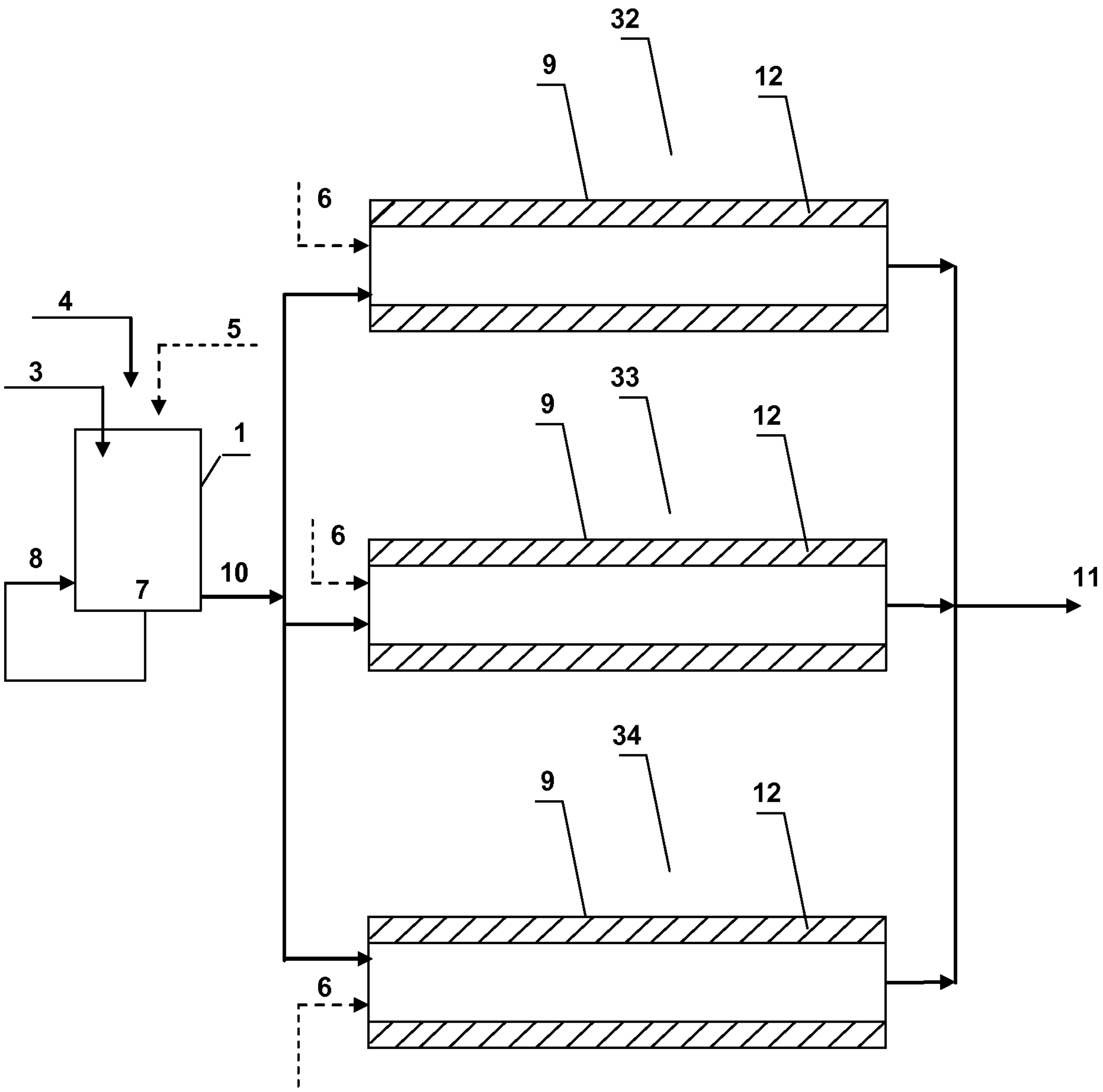


**Figure 5**

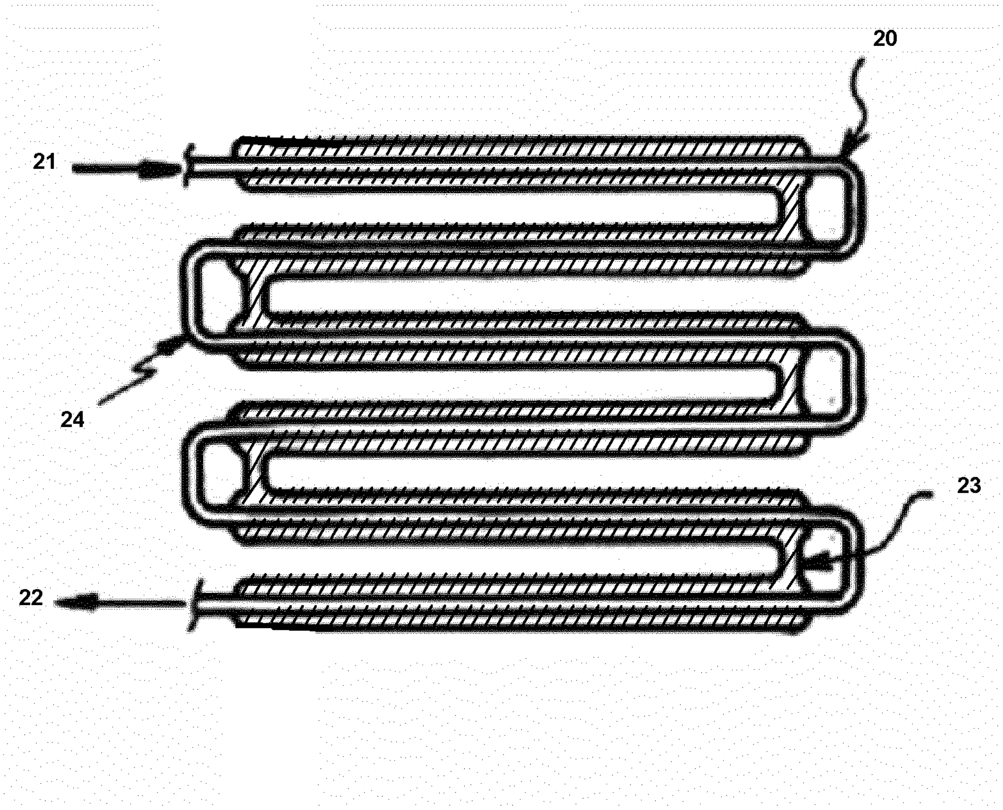


Figure 6

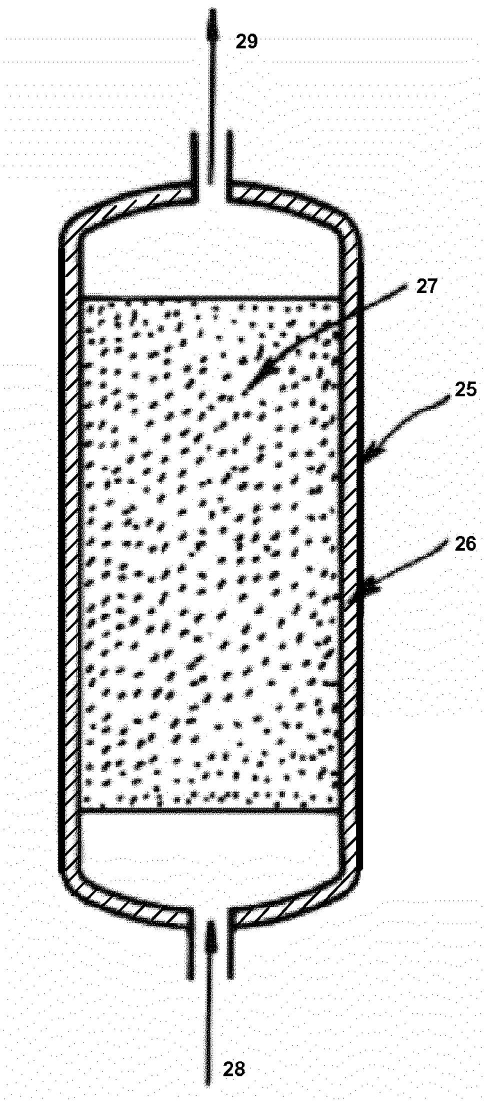


Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 50062940 A
- FR 2071464
- WO 2009150125 A
- WO 2010115784 A


### Littérature non-brevet citée dans la description


- *APPLIED CATALYSIS A: GENERAL,* 2007, vol. 327 (2), 278-286
- *CATALYSIS TODAY,* 2009, vol. 143, 145-150
- **H.C. BROWN.** Advanced Organic Chemistry. 1985, 243, , 244
- Don't Be Baffled By Static Mixers. *Chemical Engineering,* Mai 2003
- **J. VILLERMAUX.** Génie de la réaction chimique ; conception et fonctionnement des réacteurs. Tec & Doc Lavoisier, 1993
- **O. LEVENSPIEL.** Chemical Reaction Engineering. Wiley Int, 1972